# EUROPÄISCHE PATENTANMELDUNG

(11) **EP 4 374 830 A2**
(43) Veröffentlichungstag der Anmeldung: **29.05.2024**
(21) Anmeldenummer: 24168946.2
(22) Anmeldetag: 08.08.2017
(51) Int. Cl.: A61F 2/78

(54) **PROTHESENLINER**

(30) Priorität: 08.08.2016 DE 102016114681
(62) Teilanmeldung aus: 22189053.6
(71) Anmelder: Ottobock SE & Co. KGaA, 37115 Duderstadt (DE)
(72) Erfinder: Die Erfindernennung liegt noch nicht vor
(74) Vertreter: Gramm, Lins & Partner Patent- und Rechtsanwälte PartGmbB

(57) **Zusammenfassung**

Die Erfindung betrifft einen Prothesenliner, der zum Anlegen über einen Stumpf einer Gliedmaße vorgesehen ist, mit einem Grundkörper, der eine zur Haut des Stumpfes hin gerichtete Innenseite und eine von dem Stumpf weg gerichtete Außenseite aufweist, mit einer proximalen Einstiegsöffnung und einer Seitenwand, die sich von der Einstiegsöffnung bis zu einem geschlossenen distalen Endabschnitt erstreckt, wobei an der Außenseite des Grundkörpers zumindest eine Dichtlippe angeordnet ist, die von dem Grundkörper radial nach außen ragt, wobei der Umfang der Seitenwand im unbelasteten Zustand eine angenähert dreieckige Form mit abgerundeten Ecken und die Dichtlippe im unbelasteten Zustand eine angenähert dreieckige Form mit abgerundeten Ecken oder eine angenähert runde Kontur aufweist.

## Beschreibung

Die Erfindung betrifft einen Prothesenliner, der zum Anlegen über einen Stumpf einer Gliedmaße vorgesehen ist, mit einem Grundkörper, der eine zur Haut des Stumpfes hin gerichtete Innenseite und eine von dem Stumpf weg gerichtete Außenseite aufweist, mit einer proximalen Einstiegsöffnung und einer Seitenwand, die sich von der Einstiegsöffnung bis zu einem geschlossenen, distalen Endabschnitt erstreckt.

Prothesen dienen zum Ersatz fehlender Körperteile oder Gliedmaße und können auf verschiedene Arten und Weisen an einem Patienten festgelegt werden. Eine Möglichkeit der Festlegung einer Prothese, insbesondere einer Prothese einer oberen oder unteren Extremität, besteht darin, die Prothese mit einem Schaft zu versehen, der an einem Stumpf der Gliedmaße befestigt wird. Diese sogenannte Schafttechnologie ist weit verbreitet. Eine unmittelbare Anlage eines Prothesenschaftes an dem Stumpf kann problematisch sein, da der in der Regel mit einem geschlossenen Querschnitt ausgebildete, aus einem formstabilen Werkstoff bestehende Prothesenschaft zu Druckstellen oder Scheuerstellen führen kann. Darüber hinaus ist ein Volumenausgleich nicht oder nur in einem eng begrenzten Maß über den Prothesenschaft realisierbar.

Um einerseits die bei der Nutzung des Prothesenschaftes auftretenden Druckkräfte gleichmäßig zu verteilen, eine Polsterung vorzunehmen und andererseits eine Schutzschicht für die Haut des Stumpfes zur Verfügung zu stellen, sind sogenannte Prothesenliner entwickelt worden, die aus einem elastischen Werkstoff bestehen und hülsenartig mit einem geschlossenen distalen Endstück ausgebildet sind. Der Prothesenliner wird zum Anlegen herabgerollt, der Stumpf mit dem Stumpfende in das distale Endstück eingeführt und anschließend wird der Prothesenliner auf dem Stumpf abgerollt. Alternativ wird der Prothesenliner nach außen gewendet, so dass sich die eigentliche Innenseite vor dem Anlegen außen befindet. Der Prothesenliner ist so "auf links gedreht". Das Stumpfende wird auf die geschlossene Spitze des umgedrehten Prothesenliners aufgesetzt und der Prothesenliner zum Anlegen über den Stumpf gezogen. Somit erstreckt sich der Prothesenliner über einen Abschnitt des Stumpfes entlang der Hautoberfläche und dient als Zwischenstück zwischen dem Stumpf und dem Prothesenschaft. An dem distalen Endbereich des Prothesenliners können mechanische Verriegelungsmittel vorgesehen sein, die als sogenannter Pin-Lock bekannt sind. Dazu wird ein Stift in eine Aufnahmeeinrichtung in den Prothesenschaft eingeführt und dort formschlüssig verriegelt.

Eine Alternative oder eine Ergänzung zu einer formschlüssigen Verrieglung besteht in einer sogenannten Saugschafttechnologie, bei der ein geschlossener Hohlraum zwischen der Außenseite des Prothesenliners und der Innenseite eines Prothesenschaftes hergestellt und dieser Hohlraum mit einem Unterdruck versehen wird. Der Unterdruck gegenüber der Umgebung kann über eine elektrische oder mechanische Pumpe erzeugt werden, alternativ wird die Luft aus dem Zwischenraum über eine Pumpbewegung beim Gehen oder bei der Betätigung mit der Prothese durch ein Rückschlagventil herausgedrückt. Zum Ablegen der Prothese wird dann das Ventil geöffnet, Umgebungsluft kann in den Zwischenraum einströmen und der Prothesenschaft kann abgelegt werden.

Die Innenseite des Prothesenliners kann haftend ausgebildet oder mit einer haftenden Beschichtung versehen sein, beispielsweise über eine Silikonschicht, die vergleichsweise gut auf der Hautoberfläche eines Stumpfes anhaftet. Zur Erzeugung des Hohlraumes kann ein oberes Ende des Prothesenliners über das obere Ende eines Prothesenschaftes umgeschlagen werden.

Aufgabe der vorliegenden Erfindung ist es, einen Prothesenliner bereitzustellen, der gegenüber den bekannten Prothesenlinern eine verbesserte Sicherheit gegen ein Herausrutschen und einen erhöhten Tragekomfort bereitstellt.

Erfindungsgemäß wird diese Aufgabe durch einen Prothesenliner mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Der erfindungsgemäße Prothesenliner, der zum Anlegen über einen Stumpf einer Gliedmaße vorgesehen ist, mit einem Grundkörper, der eine zur Haut des Stumpfes hin gerichtete Innenseite und eine von dem Stumpf weg gerichtete Außenseite aufweist, mit einer proximalen Einstiegsöffnung und einer Seitenwand, die sich von der Einstiegsöffnung bis zu einem geschlossenen, distalen Endabschnitt erstreckt, sieht vor, dass an der Außenseite des Grundkörpers zumindest eine Dichtlippe angeordnet ist, die von dem Grundkörper radial nach außen ragt. Durch die zumindest eine radial nach außen ragende Dichtlippe ist es möglich, eine verbesserte Abdichtung und somit einen kontrollierten Hohlraum zwischen der Außenseite des Prothesenliners und der Innenseite des Prothesenschaftes bereitzustellen. Die Dichtlippe ragt im nicht in den Prothesenschaft eingeführten Zustand radial von dem Grundkörper nach außen, so dass ein gegenüber dem Grundkörper vergrößerter Außendurchmesser an der Außenseite der Dichtlippe bereitgestellt wird. Durch einen vergrößerten Außendurchmesser wird eine verbesserte Anlage an den Prothesenschaft bewirkt, so dass der Hohlraum zwischen dem Prothesenliner und dem Prothesenschaft sicher ausgebildet wird. Über diesen sicher durch die Dichtlippe abgedichteten Hohlraum ist es möglich, einen stabilen Unterdruck oder ein Vakuum innerhalb des Hohlraumes zu erzeugen, so dass während einer Bewegung der Prothese der Prothesenschaft sicher an dem Prothesenliner gehalten wird. Der Umfang der Seitenwand weist im unbelasteten Zustand eine angenähert dreieckige Form mit abgerundeten Ecken und die Dichtlippe im unbelasteten Zustand eine angenähert dreieckige Form mit abgerundeten Ecken oder eine angenähert runde Kontur auf.

Eine Weiterbildung der Erfindung sieht vor, dass die Dichtlippe einen in Richtung auf die Einstiegsöffnung geneigten Abschnitt aufweist, so dass ein sich in distalproximal-Richtung vergrößernder Durchmesser der Dichtlippe ergibt. Durch den in Richtung auf die Einstiegsöffnung geneigten Abschnitt ist es möglich, einerseits ein leichtes Einführen in den Prothesenschaft bei einem Einsteigen bereitzustellen und andererseits einen sich selbst verstärkenden Dichteffekt bei einer möglichen Auszugsbewegung zu erzeugen. Sobald sich ein Unterdruck in dem distal von der Dichtlippe ausgebildeten Hohlraum zwischen der Außenseite des Prothesenliners und der Innenseite des Prothesenschaftes ausgebildet hat, wird die Dichtlippe gegen die Innenseite des Prothesenschaftes gedrückt, wodurch sich ein selbst verstärkender Dichteffekt einstellt. Je größer der Unterdruck ist, desto höher ist der Anpressdruck, der auf der proximalen Seite der Dichtlippe anliegt.

Die Seitenwand des Grundkörpers weist bevorzugt distal zu der Dichtlippe einen geschlossenen Querschnitt auf, um zu gewährleisten, dass eine Abdichtung distal zu der Dichtlippe erfolgt. Der Querschnitt der Seitenwand kann über die gesamte Länge des Prothesenliners geschlossen sein, wodurch gewährleistet ist, dass der Prothesenliner über seine gesamte Länge vollumfänglich an dem Stumpf oder der Gliedmaße anliegt. Die Seitenwand kann in ihrer Längserstreckung geradlinig ausgebildet sein oder eine Krümmung aufweisen.

Eine Weiterbildung der Erfindung sieht vor, dass der geneigte Abschnitt im Querschnitt er Dichtlippe geradlinig oder gekrümmt ausgebildet ist. Ein gekrümmter Dichtlippenquerschnitt stellt einen vergrößerten Anlagebereich an der Innenseite des Prothesenschaftes bereit, wenn die Krümmung in Richtung auf den Grundkörper ausgebildet ist. Die Krümmung kann von dem Grundkörper weg oder in Richtung auf den Grundkörper ausgebildet sein. Bei einer Krümmung von dem Grundkörper weg verstärkt sich der Anpressdruck der Dichtlippe bei einem sich erhöhenden Unterdruck in dem geschlossenen Hohlraum oder Zwischenraum zwischen der Prothesenlineraußenseite und der Prothesenschaftinnenseite. Bei einer entgegengesetzt gerichteten Krümmung wird das Aussteigen erleichtert und gegebenenfalls eine vergrößerte Dichtfläche bereitgestellt.

An den geneigten Abschnitt der Dichtlippe kann sich ein in Richtung auf den Grundkörper gerichteter Proximalabschnitt anschließen, so dass sich ein dachartiger Querschnitt oder ein gewölbter Querschnitt im nicht angelegten Zustand des Prothesenliners einstellt. Über den in Richtung auf den Grundkörper gerichteten Proximalabschnitt kann eine Versteifung oder Stabilisierung der Dichtlippe erfolgen. Darüber hinaus kann eine Anlagekante oder Dichtkante erzeugt werden, die stabil an der Innenseite des Prothesenschaftes anliegt, wenn der Prothesenliner in den Prothesenschaft eingeführt ist.

Der Proximalabschnitt kann sich bis an die Außenseite des Grundkörpers erstrecken, so dass der Proximalabschnitt zumindest teilweise auf dem Grundkörper aufliegt oder an dem Grundkörper anliegt. Der Proximalabschnitt kann mit seinem Ende an der Außenseite des Grundkörpers aufliegen, so dass zwischen dem Grundkörper und der Dichtlippe ein Volumen umschlossen wird. Das Aufliegen des proximalen Endes der Dichtlippe auf der Außenseite des Grundkörpers vergrößert die Stabilität und erhöht einen Widerstand gegen eine Verformung der Dichtlippe beim Einführen, so dass ein erhöhter Druck der Dichtlippe gegen die Innenseite des Prothesenschaftes nach dem Einführen bereitgestellt wird.

Die Dichtlippe kann an dem Grundkörper angegossen sein. Dazu ist es vorteilhaft, wenn die Dichtlippe aus einem Material besteht, das identisch mit dem des Grundkörpers ist. Darüber hinaus ist es möglich, dass das Material der Dichtlippe nicht identisch mit dem Material des Grundkörpers ist, jedoch aus einem gleichartigen Material besteht, beispielsweise aus einem Silikon oder einem vernetzten Polymer, wobei das Material der Dichtlippe aus einem Silikon oder Polymer hergestellt sein kann, das beispielsweise härter als das Material des Grundkörpers ist. Für das Angießen der Dichtlippe an dem Grundkörper ist es wesentlich, dass sich das Material des Grundkörpers und das Material der Dichtlippe miteinander vernetzen, wobei es unschädlich ist, dass das Material des Grundkörpers beim Angießen der Dichtlippe bereits teilvernetzt sein kann. Der Grundkörper kann auch an die Dichtlippe angegossen werden.

Neben der Ausgestaltung der Dichtlippe aus dem gleichen Material wie dem Material des Grundkörpers ist es auch möglich, dass die Dichtlippe aus einem von dem Grundkörper abweichenden Material ausgebildet ist. Ein Angießen ist dann ebenfalls möglich, wenn sich beide Materialien miteinander vernetzen. Das Angießen findet in einem separaten Herstellschritt statt, der beispielsweise bei dem Angießen einer Dichtlippe nach der Ausbildung des Grundkörpers, zumindest mit einer Teilvernetzung, stattfindet.

Bei einer Ausgestaltung der Dichtlippe aus einem gleichen Material wie dem des Grundkörpers ist es in einer Variante vorgesehen, dass die Dichtlippe angeklebt oder angeschweißt wird. Neben einer Befestigung der Dichtlippe an der Außenseite des Grundkörpers über Verkleben oder Verschweißen, sowohl bei einem gleichen als auch bei einem abweichenden Material zum Grundkörper, ist es in einer Variante der Erfindung vorgesehen, dass der geneigte Abschnitt der Dichtlippe teilweise in dem Grundkörper eingebettet ist, insbesondere in dem Grundkörper eingegossen ist. Dazu wird die Dichtlippe zunächst separat hergestellt und in der Form für den Prothesenliner gehalten. Anschließend wird das Material des Grundkörpers zugeführt und um den geneigten Abschnitt der Dichtlippe teilweise herumgeleitet, so dass eine Einbettung in den Grundkörper erfolgt. Der in den Grundkörper eingebettete Teil des geneigten Abschnittes der Dichtlippe kann Löcher oder Durchbrüche aufweisen, die das Material des Grundkörpers durchdringen kann, so dass eine formschlüssige Verbindung zusätzlich zu einer gegebenenfalls stattfindenden Vernetzung mit dem Material der Dichtlippe stattfinden kann.

Die Dichtlippe ist bevorzugt umlaufend um den Grundkörper herum angeordnet, um eine vollständige Abdichtung des distal zu der Dichtlippe mit dem Prothesenschaft ausgebildeten Hohlraums zu gewährleisten. Die Dichtlippe kann eine Dichtkante ausbilden, die in einer Ebene senkrecht zu der Längserstreckung des Prothesenliners liegt. Grundsätzlich ist es auch möglich, die Dichtkante oder den radial äußeren Bereich der Dichtlippe in einer Ebene verlaufen zu lassen, die geneigt zu der Längserstreckung des Prothesenliners orientiert ist.

Neben einer integralen Ausgestaltung der Dichtlippe zusammen mit dem Grundkörper ist es möglich, dass die Dichtlippe als separates Bauteil ausgebildet ist und an dem Grundkörper befestigt, eingegossen oder angegossen wird.

Die Dichtlippe ist proximal zu dem distalen Endbereich des Prothesenliners angeordnet, insbesondere proximal zu dem ersten Drittel des Grundkörpers, so dass zumindest ein Drittel der Länge des Prothesenliners genutzt wird, um einen Hohlraum mit dem Prothesenschaft auszugestalten. Um eine erhöhte Haltekraft über den Unterdruck bereitzustellen, ist in einer Weiterbildung der Erfindung vorgesehen, dass die Dichtlippe in der Mitte des Grundkörpers, also auf der Hälfte der Länge des Prothesenliners oder aber proximal dazu an dem Grundkörper angeordnet oder daran befestigt ist.

Eine Weiterbildung der Erfindung sieht vor, dass die Außenseite des Prothesenliners textillos ausgebildet ist, also aus dem Grundmaterial des Grundkörpers besteht. Alternativ zu einer Ausgestaltung der Außenseite des Prothesenliners aus dem Material des Grundkörpers ist vorgesehen, dass die Außenseite mit einer reibungsvermindernden oder reibungsminimierenden Beschichtung versehen ist, über die eine Veränderung der chemischen und physikalischen Eigenschaften der Außenseite erreicht werden kann. Die Beschichtung kann beispielsweise als Parylenbeschichtung in einem CVD-Verfahren. Grundsätzlich ist es auch möglich, eine bereichsweise Beschichtung der Außenseite, beispielsweise über das CVD-Verfahren, durchzuführen, so dass die Außenseite des Prothesenliners mit bereichsweise unterschiedlichen Eigenschaften versehen werden kann. Beispielsweise kann der Bereich proximal der Dichtlippe mit einer glatten Beschichtung versehen werden, so dass eine Relativbewegung des Prothesenliners proximal der Dichtlippe zu dem Prothesenschaft leicht erfolgen kann, während der distal der Dichtlippe befindliche Bereich der Außenseite des Prothesenliners eine besonders gute Haftfähigkeit an der Innenseite des Prothesenschaftes aufweist. Bei einer alternativen Ausgestaltung ist es möglich, dass die Außenseite des Prothesenliners distal zu der Dichtlippe besonders glattwandig ausgebildet ist, um so eine Relativbewegung und damit einen Pumpeffekt zwischen der Innenseite des Prothesenschaftes und der Außenseite des Prothesenliners zu ermöglichen. Über eine haftende Ausgestaltung der Oberfläche proximal zu der Dichtlippe wird eine erhöhte Anhaftung des Prothesenliners an dem Prothesenschaft bewirkt, um ein verbessertes Stabilitätsempfinden für den Nutzer des Prothesenliners bereitzustellen. Ebenfalls kann die Außenseite des Prothesenliners mit einer Matrix, beispielsweise einem Gitter versehen sein, das zur Verstärkung des Grundkörpers und zur Bereitstellung eines erhöhten Widerstandes gegen ein Einreißen oder Weiterreißen des Grundkörpers bei einer Beschädigung dient.

Die Außenseite des Prothesenliners kann zumindest teilweise mit einer aufgerauten Oberflächenstruktur versehen sein, die insbesondere distal zu der Dichtlippe ausgebildet ist, um ein Anhaften des distal zu der Dichtlippe befindlichen Linerabschnittes an der Oberfläche des Prothesenschaftes zu vermeiden. Die Oberflächenstruktur erleichtert insbesondere die Vakuumverteilung. Hierzu können Mikrostrukturen in der Oberfläche vorhanden sein, die die Verteilung erleichtern und untereinander verbunden sind. Die Oberflächenstruktur sichert insbesondere die Verteilung des Vakuums bis zu der Dichtlippe. Sofern ein Schaftventil vorhanden ist, wäre ein Abdichtung in unmittelbarer Nähe zu dem Schaftventil bei einer sehr glatten Ausgestaltung der Außenseite des Liners problematisch, da dann nur ein sehr kleiner Bereich mit Vakuum beaufschlagt werden würde. Durch das Nichtanhaften der Außenseite des Prothesenliners im distalen Bereich der Dichtlippe oder die Strömungskanäle an der Außenseite kann ein leichtes Ablösen der Außenseite des Prothesenliners von der Innenseite des Prothesenschaftes und eine Druckverteilung erfolgen, wodurch ein erneuter Unterdruck oder ein gleichmäßiges Vakuum bis zur Dichtlippe bereitgestellt und damit ein sicheres Halten des Prothesenschaftes an dem Prothesenliner ermöglicht wird.

Eine Weiterbildung der Erfindung sieht vor, dass an der Innenseite und/oder Außenseite des Prothesenliners Streifen aus einem von dem Material des Grundkörpers abweichenden Material angeordnet sind. Die unterschiedlichen Materialien zeichnen sich durch unterschiedliche chemische und/oder physikalische Eigenschaften aus, insbesondere können dadurch bereichsweise vergrößerte oder verminderte Haftfähigkeiten, Elastizitäten und/oder Färbungen erzielt werden. Die unterschiedlichen Materialien können durch Additive in einem Grundmaterial, aus dem der Grundkörper ebenfalls besteht, erzeugt werden. Statt die Streifen auf der Basis eines gleichen Grundmaterials wie das Material des Grundkörpers herzustellen, können auch abweichende Materialien oder andere Elastomertypen in der Außenseite und/oder Innenseite des Prothesenliners eingebettet oder eingegossen sein. Die Streifen können auch aufgerakelt sein. Die Streifen können geradlinige oder kurvenartige Konturen aufweisen und beabstandet zueinander an der Außenseite und/oder Innenseite des Grundkörpers angeordnet sein.

In einer Weiterbildung der Erfindung ist vorgesehen, dass in dem Grundkörper zumindest eine Matrix mit isotropen Eigenschaften, insbesondere isotropen Elastizitäten, eingebettet ist. Die Matrix dient insbesondere als Verstärkungsmatrix und verhindert beispielsweise ein Einreißen oder Weiterreißen bei einem teilweise beschädigten Prothesenliner. Die Matrix kann korrespondierend zu dem Prothesenliner ausgebildet sein und sowohl im distalen Endbereich als auch in der Seitenwand angeordnet und von dem Material des Grundkörpers umgeben sein. Ebenfalls ist es möglich, dass der distale Endbereich des Prothesenliners nicht mit einer Matrix versehen ist, jedoch die Seitenwand die Matrix aufweist. Die Matrix kann als umfänglich geschlossene Hülse ausgebildet sein. Die Matrix kann sich bis zu dem proximalen Rand des Prothesenliners, der sich um die proximale Einstiegsöffnung herum erstreckt, erstrecken. Am proximalen Rand muss die Matrix nicht von dem Material des Grundkörpers umgeben sein.

Eine Weiterbildung der Erfindung sieht vor, dass mehrere Matrizes getrennt voneinander in dem Grundkörper eingebettet sind. Beispielsweise können die Matrizes streifenförmig in Längserstreckung des Prothesenliners in der Seitenwand eingearbeitet sein, wobei die Matrizes isotrope Elastizitäten aufweisen, wobei das Material der jeweiligen Matrix eine geringere Elastizität als das Material des Grundkörpers aufweist. Dadurch ist es möglich, eine Dehnung in Längserstreckung des Prothesenliners zu begrenzen, durch die Zwischenräume zwischen den Matrizes jedoch einen Volumenausgleich zu ermöglichen bzw. eine Volumenänderung auszugleichen. Dadurch wird einerseits eine ausreichende Längsstabilität gegen den sogenannten Melkeffekt über die Matrizes erreicht, ohne andererseits die Elastizität in Umfangsrichtung zum Ausgleich von Volumenschwankungen unverhältnismäßig einzuschränken. Die Streifen können geradlinige oder kurvenartige Konturen aufweisen und beabstandet zueinander an der Außenseite und/oder Innenseite des Grundkörpers angeordnet sein. Die Wellenform dient dazu, einen evt. auftretenden Faltenwurf in definierte Bahnen zu lenken, so dass man viele minimale, nicht störende Falten erhält.

Eine Weiterbildung der Erfindung sieht vor, dass mehrere Matrizes mit unterschiedlichen Elastizitäten in dem Grundkörper eingebettet sind. So kann ein Typ einer Matrix in mehreren Streifen beabstandet zueinander in dem Grundkörper eingebettet sein, wobei das Material des jeweiligen Matrixstreifens eine Dehnung im Wesentlichen unterbindet. Im Bereich der Zwischenräume zwischen den Matrizes des ersten Materialtyps können Matrizes aus einem Material mit einer vergleichsweise hohen Elastizität, insbesondere einer Elastizität, die gleich oder größer dem des Grundmaterial des Grundkörpers ist, eingebettet sein. Dieses Material kann jedoch eine erhöhte Reißfestigkeit oder Schnittfestigkeit im Vergleich zu dem Material des Grundkörpers aufweisen, so dass einerseits eine Dehnung längs der Längserstreckung des Prothesenliners über die Matrizen des ersten Materialtyps verhindert wird, eine Elastizität in Umfangsrichtung über das Material der zweiten Matrizen jedoch nicht eingeschränkt wird. Darüber wird hinaus eine erhöhte Reißfestigkeit durch die eingebetteten Materialien der beiden Materialtypen bereitgestellt.

Die erste unelastische Matrix kann in Umfangsrichtung beabstandet zu einer elastischen zweiten Matrix in dem Grundkörper eingebettet sein, grundsätzlich ist es auch möglich, dass sich die beiden Matrizen in Umfangsrichtung teilweise überlappen. Es ist auch möglich, dass Streifen aus einem flexiblen, unelastischen Material als erste Matrix beabstandet zueinander in Umfangsrichtung in dem Grundkörper eingebettet ist und die zweite Matrix aus einem flexiblen und elastischen Material hülsenförmig mit einem geschlossenen Umfang in dem Grundmaterial radial beabstandet zu der ersten Matrix oder den Streifen eingebettet ist. Die Matrizen können durch das Grundmaterial voneinander getrennt sein oder auch teilweise oder vollständig aneinander anliegen.

Die Matrizen können bevorzugt ausschließlich medial und lateral zu einem natürlichen Gelenk in einem angelegten Zustand verlaufen, wodurch die Beugung des Gelenkes, insbesondere des Kniegelenkes nicht oder minimal beeinflusst wird. Die Streifen verlaufen insbesondere in dem Bereich des Kompromissdrehpunktes des Gelenkes.

In dem Grundkörper kann eine Aufnahme für einen Pumpenanordnung angeordnet sein, mit der Luft aus dem Hohlraum, der von der Dichtlippe, der Innenseite des Prothesenschaftes und der Außenseite des Prothesenliners abgegrenzt wird, oder aus dem Innenraum des Prothesenliners, in dem sich der Stumpf befindet, heraus befördert wird. Die Pumpenanordnung kann zusätzlich zu dem Pumpeffekt beim Belasten und Entlasten des Prothesenliners bei der Benutzung der Prothese eingesetzt werden. Dabei ist die Aufnahme für die Pumpenanordnung bevorzugt an dem distalen Ende des Grundkörpers angeordnet.

Bevorzugt ist der Grundkörper des Prothesenliners aus einem Elastomermaterial ausgebildet, insbesondere aus einem Silikon oder einem Polymer wie thermoplastisches Copolymer oder Polyurethan.

Der Grundkörper kann aus einem luftdurchlässigen Material bestehen, zumindest teilweise, und ist distal abgedichtet. Auf der Außenseite des Grundkörpers kann eine Dichtkappe aus einem luftundurchlässigen Material angeordnet sein, um eine distale Abdichtung zu erreichen.

Nachfolgend werden Ausführungsbeispiele der Erfindung anhand der beigefügten Figuren näher erläutert. Es zeigen:
- Figur 1 -: eine Seitenansicht eines Prothesenliners;
- Figur 2 -: eine Schnittansicht des Prothesenliners gemäß Figur 1 ;
- Figur 3 -: eine Schnittansicht einer Variante eines Prothesenliners;
- Figur 4 -: eine weitere Variante des Prothesenliners mit einer proximal verschobenen Dichtlippe;
- Figur 5 -: eine Variante der Figur 4 mit einer eingebetteten Matrix;
- Figur 6 -: eine Schnittdarstellung des Prothesenliners mit einer Pumpenaufnahme;
- Figur 7 -: eine Variante der Figur 6;
- Figur 8 -: eine Variante der Figur 5 mit einer distalen Dichtlippe und einer hülsenförmigen Matrix;
- Figur 9 -: eine Schnittdarstellung durch einen Prothesenliner mit einer proximalen Dichtlippe;
- Figur 10 -: eine Detailschnittdarstellung eines Prothesenliners mit einer angegossenen Dichtlippe;
- Figur 11 -: eine Variante der Figur 10 mit einer eingegossenen Dichtlippe;
- Figur 12 -: eine Schnittdarstellung eines Prothesenliners mit eingebetteten Streifen auf der Innenseite;
- Figur 13 -: eine Seitenansicht eines Prothesenliners mit Streifen auf der Außenseite;
- Figur 14 -: eine Seitenansicht eines Prothesenliners mit eingebetteten Streifen einer Matrix;
- Figur 15 -: eine Seitenansicht eines Prothesenliners mit aufgerauter distaler Oberfläche;
- Figuren 16a bis 16c -: eine vergrößerte Detailansicht der Außenseite im distalen Endbereich;
- Figur 17-: eine perspektivische Darstellung eines Prothesenliner mit einem distalen Pumpenkolben;
- Figur 18-: eine Schnittdarstellung der Figur 17;
- Figur 19-: eine Variante der Erfindung mit medial-lateral angeordneten Matrizes;
- Figur 20-: ein System aus Prothesenliner und Prothesenschaft in Schnittdarstellung;
- Figur 21 -: eine Variante mit einer distalen Dichtkappe;
- Figur 22 -: eine Variante der Figur 3 mit einer distal angeordneten Dichtlippe und einem flektierten Liner;
- Figur 23 -: eine Variante der Figur 22;
- Figur 24 -: eine Variante der Figur 21;
- Figur 25 -: eine Variante der Figur 24 mit einem Pin;
- Figur 26 -: eine Draufsicht auf einen Liner gemäß Figur 3; sowie
- Figur 27 -: eine Variante der Figur 26.
- Figur 28 -: eine Schnittdarstellung durch einen mehrlagigen Prothesenliner;
- Figur 29 -: eine Variante der Figur 28;
- Figur 30 -: eine Variante der Figur 29; sowie
- Figur 31 -: eine Darstellung eines Unterschenkelliners im Horizontalschnitt.

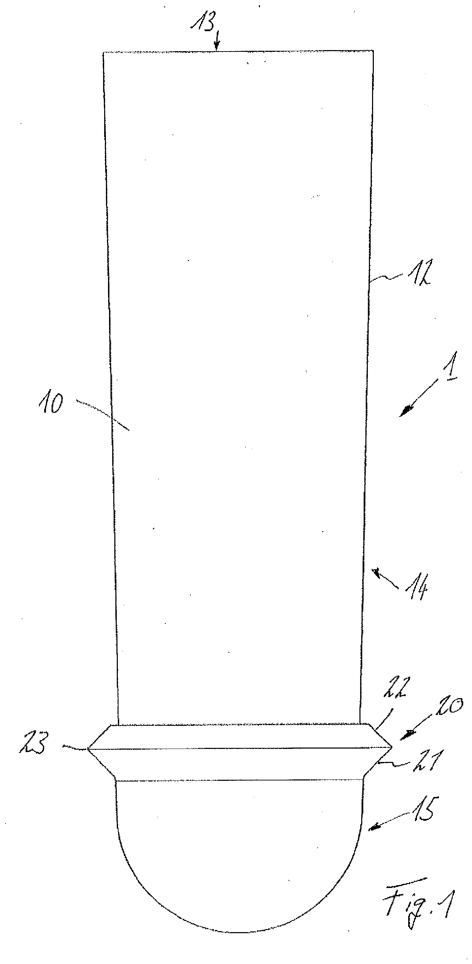

Figur 1 zeigt in einer Seitenansicht einen Prothesenliner 1, der zum Anlegen über einen Stumpf einer Gliedmaße vorgesehen ist. Der Prothesenliner 1 ist insbesondere zum Anlegen an einen Oberarmstumpf oder einen Oberschenkelstumpf vorgesehen. Der Prothesenliner 1 hat einen Grundkörper 10, der eine zur Haut des Stumpfes hingerichtete Innenseite und eine vom Stumpf weg gerichtete Außenseite 12 aufweist. An seinem proximalen Ende ist eine Einstiegsöffnung 13 vorhanden und an seinem distalen Ende ist ein geschlossener distaler Endabschnitt 15 ausgebildet, von dem aus sich eine Seitenwand zu der proximalen Einstiegsöffnung 13 erstreckt. Der Prothesenliner 1 ist innen hohl und weist entweder eine an die Form des Stumpfes angepasste oder eine zylindrische oder kegelstumpfförmige Innenkontur mit jeweils einem geschlossenen distalen Endabschnitt auf. Der Prothesenliner 1 ist somit als einseitig offener Hohlkörper ausgebildet. Bevorzugt besteht der Grundkörper 10 aus einem Elastomer, beispielsweise Polypropylen oder Polyethylen, oder Silikon.

Am Ende des distalen Endbereiches 15 ist an der Außenseite 12 des Grundkörpers 10 eine Dichtlippe 20 angeordnet, die von dem Grundkörper 10 nach außen wegragt, so dass der größte Umfang der Dichtlippe 20 größer als der Umfang des Grundkörpers 10 im Bereich der Dichtlippe 20 ist.

Die Dichtlippe 20 weist einen in Richtung auf die Einstiegsöffnung 13 geneigten Abschnitt 21 auf, der sich von dem Grundkörper 10 nach außen weg erstreckt. Der geneigte Abschnitt 21 mündet in eine äußere Dichtkante 23, von der aus sich ein in Richtung auf den Grundkörper 10 gerichteter Proximalabschnitt 22 der Dichtlippe 20 anschließt. Die Außenkontur der Dichtlippe 20 ist in dem Ausführungsbeispiel gemäß Figur 1 somit dachförmig oder winkelförmig ausgebildet, sowohl der geneigte Abschnitt 21 als auch der Proximalabschnitt 22 sind geradwandig im dargestellten Zustand ausgebildet, also in dem Zustand, in dem der Prothesenliner 1 nicht in einen nicht dargestellten Prothesenschaft eingeführt ist. Wird der Prothesenliner 1 in einen Prothesenschaft eingeführt, legt sich zunächst die Dichtkante 23 an die Innenseite des Prothesenschaftes an. Wird der Prothesenliner 1 weiter in den Prothesenschaft eingeführt, führt dies in der Regel dazu, dass die Dichtlippe 20 und insbesondere die Dichtkante 23 in Richtung auf den Grundkörper 10 gedrückt wird, wodurch der Proximalabschnitt 22 in Proximalrichtung in Richtung auf die Einstiegsöffnung 13 verlagert wird.

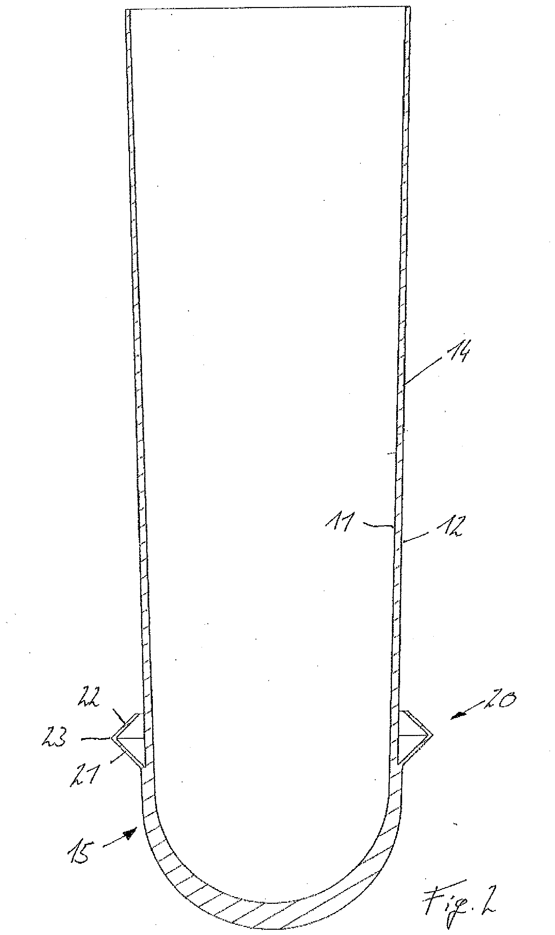

Figur 2 zeigt den Prothesenliner 1 gemäß Figur 1 in einer Schnittdarstellung. Der Figur 2 ist zu entnehmen, dass der distale Endabschnitt 15 eine größere Wandstärke als die Seitenwand 14 hat. An dem Ende des distalen Endabschnitts 15 ist die Dichtlippe 20 angeformt oder angegossen. Die Dichtlippe 20 ist somit entweder einstückig zusammen mit dem Prothesenliner 1 geformt oder als separates Bauteil ausgebildet und anschließend an dem Grundkörper 10 des Prothesenliners 1 befestigt, beispielsweise durch Ankleben oder Anschweißen. Alternativ kann die Dichtlippe 20 an dem Grundkörper 10 angegossen werden.

Die Dichtlippe 20 besteht im dargestellten Ausführungsbeispiel aus dem Material des Grundkörpers 10, weist also eine identische Materialzusammensetzung wie der Grundkörper 10 auf. Grundsätzlich ist es auch möglich, die Dichtfläche 20 an der Außenseite und/oder Innenseite zu beschichten, beispielsweise mit einer CVD-Beschichtung zu versehen, um die Eigenschaften der Dichtlippe 20 zu modifizieren, beispielsweise eine erhöhte Haftfähigkeit oder eine erhöhte Reibungsbeständigkeit bereitzustellen.

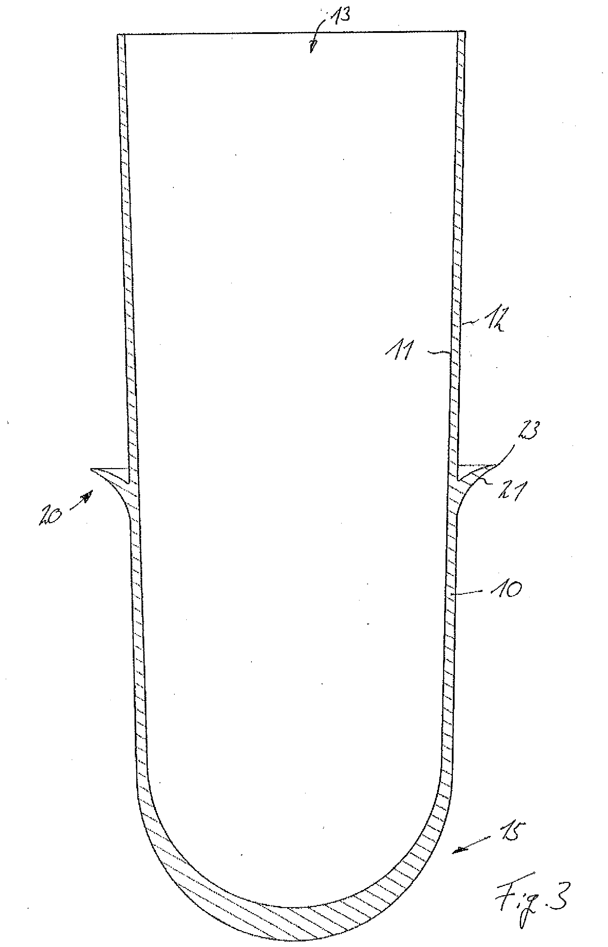

Eine Variante der Erfindung ist in der Figur 3 dargestellt, die einen Prothesenliner 1 in einer Schnittdarstellung zeigt. Auch hier weist der distale Endbereich 15 eine größere Materialstärke verglichen mit der übrigen Seitenwand 14 auf. Der erhöhte Materialeinsatz im Bereich des distalen Endbereiches 15 dient einer verbesserten Polsterung und einer Verteilung von Druckkräften, insbesondere beim Einsatz als Prothesenliner für eine Oberschenkelschaft oder Unterschenkelschaft, da bei Prothesen der unteren Extremität sehr hohe Druckkräfte während des Stehens oder Gehens auftreten. Die Dichtlippe 20 weist anders als bei der Ausführungsform gemäß der Figuren 1 und 2 einen gekrümmten Querschnitt auf, wobei die Krümmung so ausgebildet ist, dass sich der geneigte Abschnitt 21 von dem Grundkörper 10 wegerstreckt. Von distal außen betrachtet, ist der geneigte Abschnitt somit konkav gekrümmt. Der geneigte Abschnitt 21 verjüngt sich zur Außenseite hin, sodass eine vergleichsweise scharfkantige Dichtkante 23 am radial äußeren Ende des geneigten Abschnitts 21 der Dichtlippe 20 entsteht. Von der Dichtkante 23 verläuft die proximale Kontur der Dichtlippe 20 wieder distal in Richtung auf den Grundkörper 10 und die Seitenwand 14, so dass zwischen der Dichtkante 23 und der Außenseite 12 des Grundkörpers 10 ein Querschnitt angenähert dreieckiger Freiraum entsteht, der vollständig oder nahezu ausgefüllt wird, wenn der Prothesenliner 1 vollständig in einen Prothesenschaft eingeführt wird. Sofern die Dichtkante 23 nicht so weit in Richtung auf den Grundkörper 10 komprimiert wird, dass sie auf der Außenseite 12 anliegt, wird ein Spalt oder ein keilförmiger Freiraum ausgebildet, an dem der Atmosphärendruck angreifen kann. Dadurch wird die Dichtlippe 20 bei Ausbildung eines Unterdruckes in dem Hohlraum, der sich zwischen einem Prothesenschaft und dem Prothesenliner 1 bildet und von der Dichtlippe 20 abgetrennt wird, an den Prothesenschaft gedrückt, wodurch eine Dichtungsverstärkung bei einem auftretenden Unterdruck im distalen Bereich der Dichtlippe 20 entsteht. Die Dichtlippe 20 ist in dem dargestellten Ausführungsbeispiel der Figur 3 ungefähr auf Höhe der Mitte des Prothesenliners 1 angeordnet, also im Vergleich zu dem Ausführungsbeispiel gemäß der Figuren 1 und 2 wesentlich weiter proximal in Richtung auf die Einstiegsöffnung 13 gerichtet.

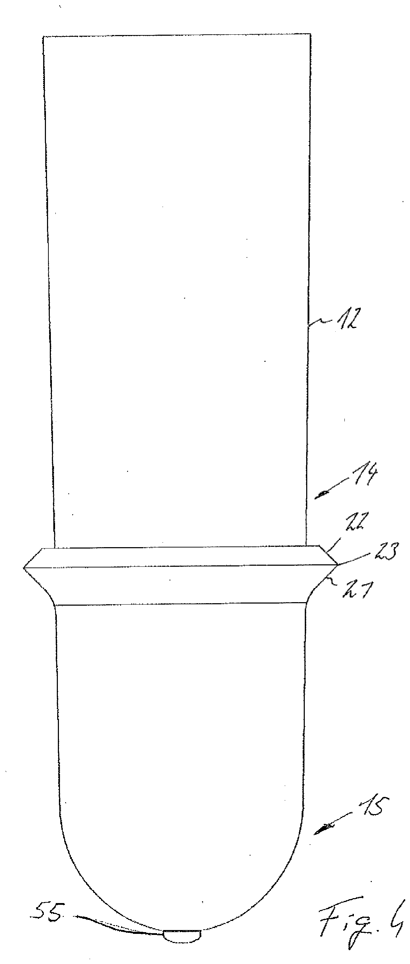

Figur 4 zeigt eine Variante der Figur 1 mit einer Dichtlippe 20, die im Vergleich zu dem Ausführungsbeispiel gemäß Figur 1 weiter proximal angeordnet ist, im darstellten Ausführungsbeispiel ist die Dichtlippe 20 proximal des ersten Drittels der Länge des Prothesenliners 1, von dem distalen Ende aus gesehen, angeordnet. An dem distalen Ende des Prothesenliners 1 ist eine Pumpenanordnung 50 angeordnet, über die Luft oder Feuchtigkeit aus dem Innenraum des Prothesenliners 1 herausgesaugt werden kann, um eine verbesserte Anhaftung der Innenseite 11 des Prothesenliners 1 an dem nicht dargestellten Stumpf zu bewirken.

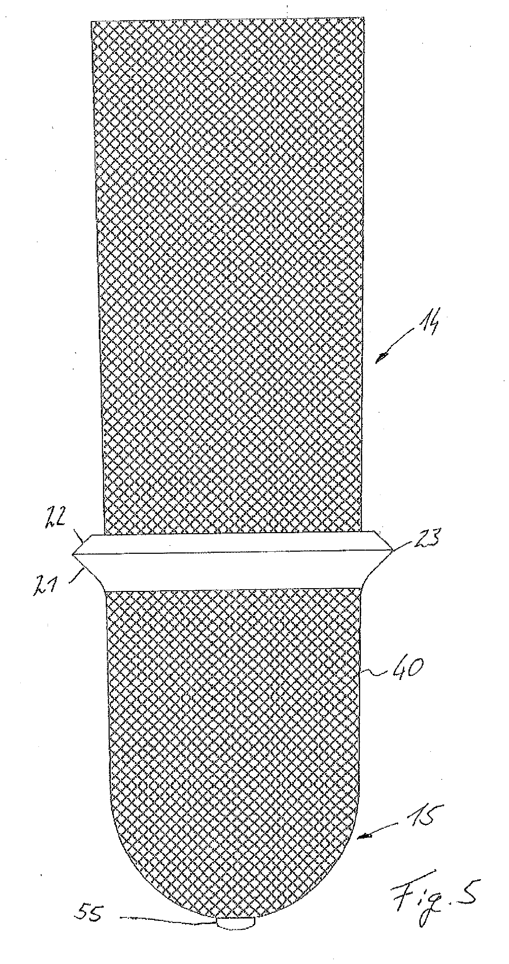

Figur 5 zeigt eine Variante der Anordnung gemäß Figur 4 mit einer Matrix 40, die in dem Grundkörper 10 eingebettet ist. Die Matrix 40 korrespondiert in der Formgebung mit der Formgebung des Grundkörpers 10 und weist eine hülsenartige Seitenwand und einen geschlossenen distalen Endbereich auf. Der Grundkörper 10 umgibt die Matrix 40 vollständig, der Matrix 40 kann gegebenenfalls im Bereich der proximalen Einstiegsöffnung 13 aus dem Grundkörpermaterial herausragen oder damit abschließen. Alternativ ist die Matrix 40 an der Außenseite 12 oder Innenseite des Grundkörpers 10 aufgebracht und dient insbesondere als Verstärkungsmaterial, unabhängig von dem Ort der Befestigung in oder auf dem Grundkörper 10.

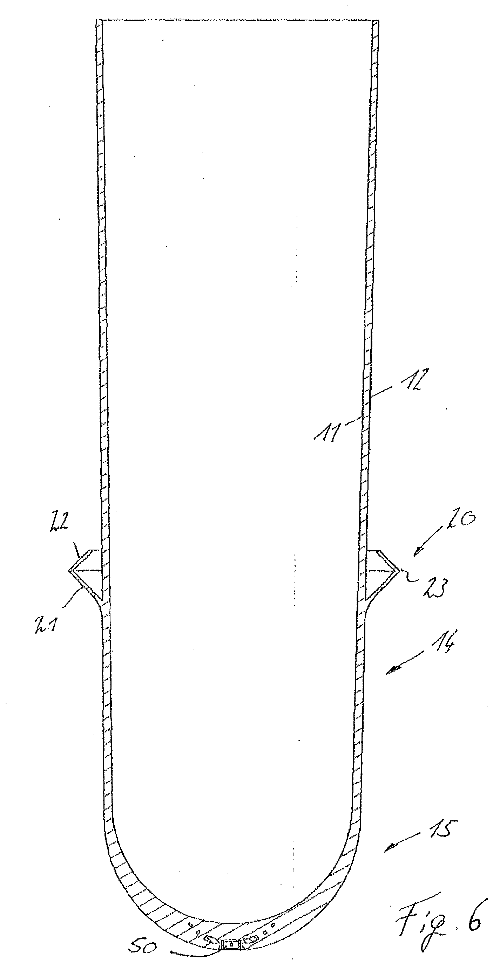

Figur 6 zeigt eine Schnittdarstellung gemäß der Figur 4, jedoch ohne Pumpenanordnung. In der Schnittdarstellung ist eine Aufnahme 50 für die Pumpenanordnung 55 gezeigt. Von der Aufnahme 50 sind Kanäle in Richtung auf den Innenraum des Prothesenliners 1 angedeutet, aus dem Luft und/oder Feuchtigkeit abgesaugt und aus dem Prothesenliner 1 heraus transportiert werden können. Zudem sind in dem distalen Ende Verstärkungselemente oder ein Anschlussteller vor die Pumpenelemente oder eine Verriegelungseinrichtung angeordnet.

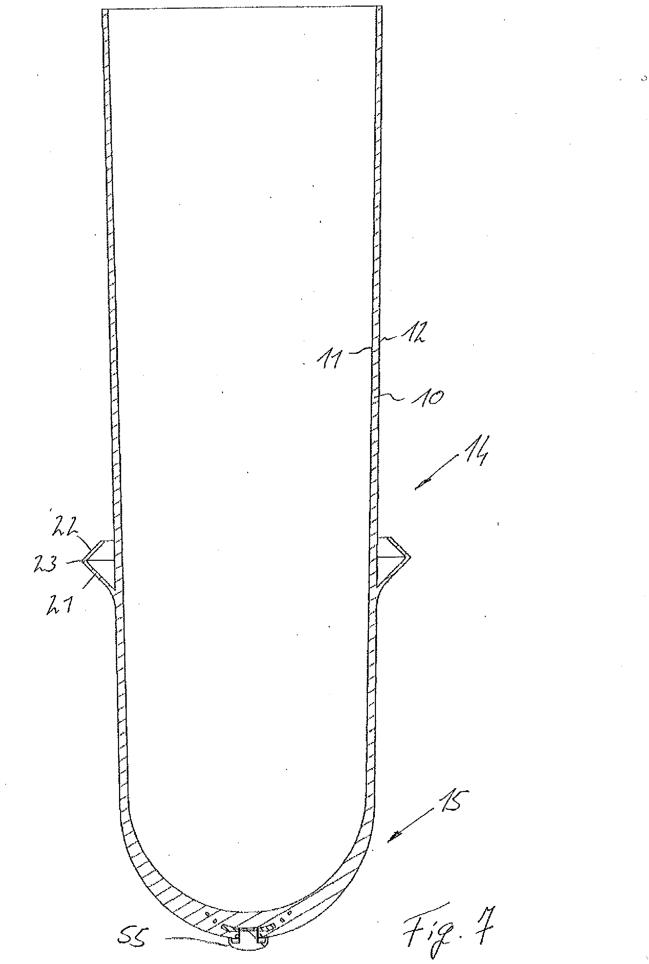

In der Figur 7 ist in der Schnittdarstellung ein Teil einer Pumpanordnung 55 in der Aufnahme 50 gezeigt. In der Aufnahme ist ein Pin 55 eingeschraubt, über den eine Verbindung zu der eigentlichen Pumpe hergestellt wird, die nicht gezeigt ist. Durch Axialbelastung in Richtung der Längserstreckung des Prothesenliners wird der Pumpmechanismus betätigt, sodass Luft aus dem Innenraum eines nicht dargestellten Prothesenschaftes herausgesaugt und abtransportiert werden kann.

Die Dichtlippe 20 weist, analog zu der Ausgestaltung gemäß der Figuren 1, 2 sowie 4 bis 6 einen Proximalabschnitt 22 auf, der im dargestellten Ausführungsbeispiel vor der Außenseite 12 der Seitenwand 14 endet, so dass ein Ringspalt von der Seitenwand 14 bis zu dem proximalen Rand der Dichtlippe 20 um den Grundkörper 10 herum ausgebildet ist. In einer Variante der Erfindung kann der Proximalabschnitt 22 sich bis an die Oberfläche der Außenseite 12 der Seitenwand 14 erstrecken, ohne mit dem Grundkörper verbunden zu sein. Die Dichtlippe 20 ist nur distal an dem Grundkörper 10 befestigt oder daran angegossen oder eingegossen. Bei einem radial nach innen wirkenden Druck wird die Dichtkante 23 nach innen in Richtung auf den Grundkörper 10 verlagert, dadurch wird der proximale Rand der Dichtlippe 20 auf der Außenseite 12 des Grundkörpers 10 nach oben verschoben. Über die Rückstellkraft der elastischen Dichtlippe 20 wird dadurch eine erhöhte Anpresskraft an den nicht dargestellten Prothesenschaft bereitgestellt.

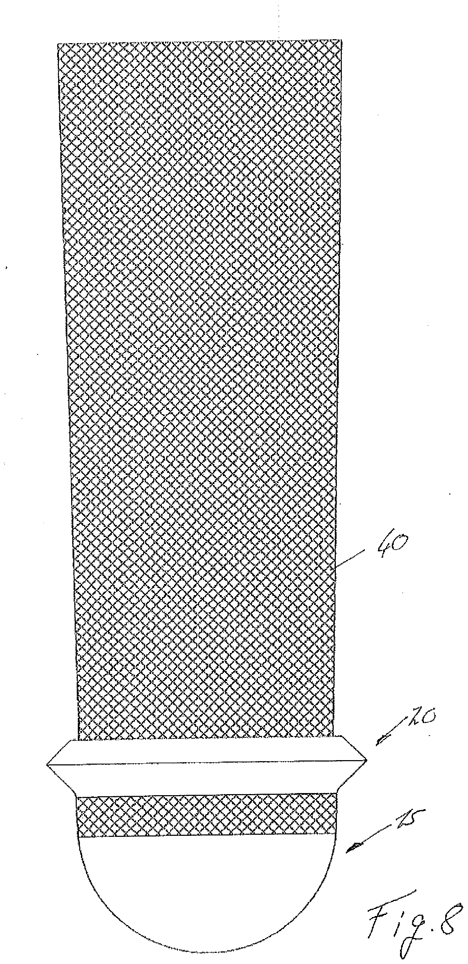

Figur 8 zeigt eine Variante der Figur 5, bei der die Dichtlippe 20 weiter distal angeordnet ist. Darüber hinaus ist der distale Endbereich 15 nicht mit der Matrix 40 versehen. Die Matrix 40 ist bei einer umlaufenden Ausgestaltung elastisch ausgebildet, so dass eine radiale Aufweitung der Seitenwand 14 erfolgen kann. Das Material der Matrix 40 ist flexibel und insbesondere so reißfest, dass bei einer Beschädigung des Grundkörpers 10, der aus einem Elastomermaterial oder Silikon besteht, eine Weiterreißneigung vermindert wird. Die Matrix 40 ist netzartig, gitterartig oder textilartig ausgebildet und weist Freiräume auf, die von dem Material des Grundkörpers 10 durchdrungen werden. Die Matrix 40 kann vollständig in dem Grundkörper 10 eingebettet sein, so dass kein Kontakt zur Außenseite 12 oder zur Innenseite 11 besteht. Alternativ kann zumindest bereichsweise die Matrix 40, insbesondere wenn sie kein Textil ist, auch auf der Außenseite 12 des Grundkörpers 10 angeordnet sein, so dass sie auf dem Grundkörper 10 aufliegt und nur teilweise darin einsinkt, um dort festgelegt zu werden. Die Matrix 40 kann vollständig oder teilweise in dem Grundkörper 10 eingebettet sein, alternativ ist die Matrix 40 auf dem Grundkörper 10 aufgeklebt, aufgerakelt oder damit verschweißt.

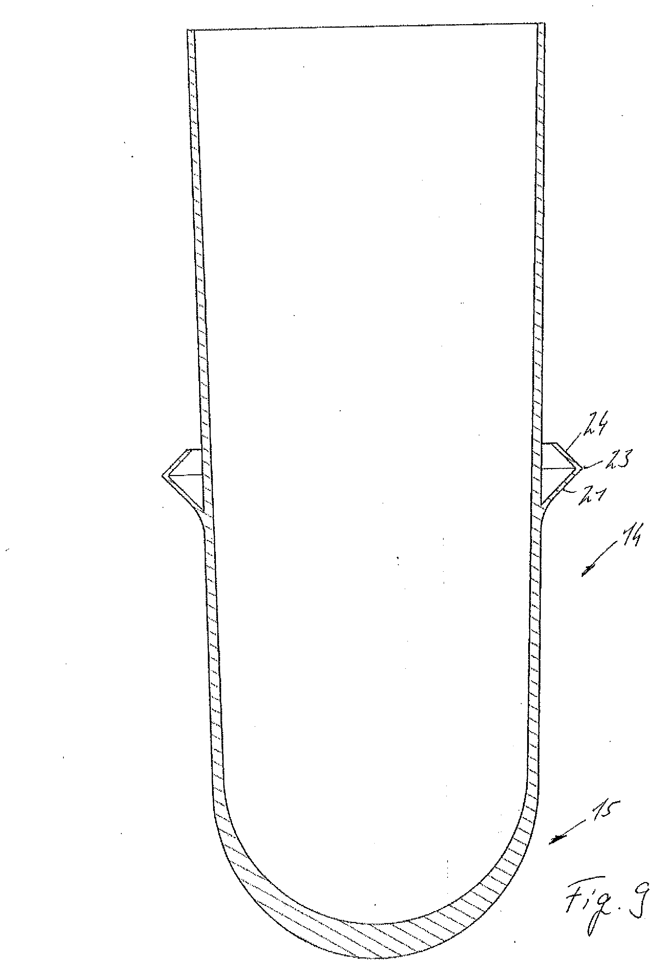

In der Figur 9 ist eine Variante der Erfindung gemäß der Figur 7 gezeigt, bei der die Dichtlippe 20 weiter proximal angeordnet ist, ungefähr in der Mitte der Länge des Prothesenliners 1. Im distalen Endbereich 15 ist kein Pin oder eine Verstärkungsplatte oder dergleichen, z.B. für eine Pumpenanordnung, angeordnet.

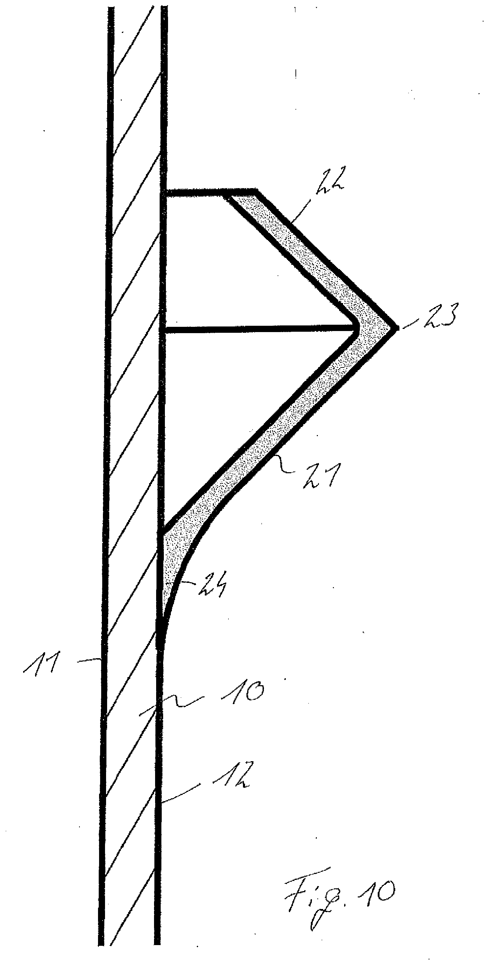

Figur 10 zeigt die Dichtlippe 20 an dem Grundkörper 10. Die Dichtlippe 20 sieht einen schräg in Richtung auf die Einstiegsöffnung 13 geneigten Abschnitt 21, eine radial proximal nach außen abstehende Dichtkante 23 sowie einen sich proximal in Richtung auf den Grundkörper von der Dichtkante 23 erstreckenden Proximalabschnitt 22 vor. Der Proximalabschnitt 22 endet vor dem Grundkörper 10, so dass ein Ringspalt und Freiraum zwischen dem proximalen Ende der Dichtlippe 20 und dem Grundkörper 10 gebildet wird. Die Dichtlippe 20 schließt einen Hohlraum ein und ist an den Grundkörper 10 angegossen. Dazu kann der Grundkörper 10 zunächst teilvernetzt werden, anschließend wird eine Angussform um den Grundkörper 10 herum angelegt und die Dichtlippe 20 angegossen, so dass in dem Kontaktbereich an dem Fuß 24 der Dichtlippe 20 eine Vernetzung zwischen dem Material der Dichtlippe 20 und dem Material des Grundkörpers 10 stattfindet. Die beiden Materialien können identisch sein, es besteht auch die Möglichkeit, dass unterschiedliche Materialien oder Materialvarianten über Hinzufügung von Additiven eingesetzt werden, um den unterschiedlichen Anforderungen an den Grundkörper 10 und der Dichtlippe 20 gerecht zu werden. Der Grundkörper 10 kann beispielsweise eine geringere Elastizität als die Dichtlippe 20 aufweisen oder umgekehrt.

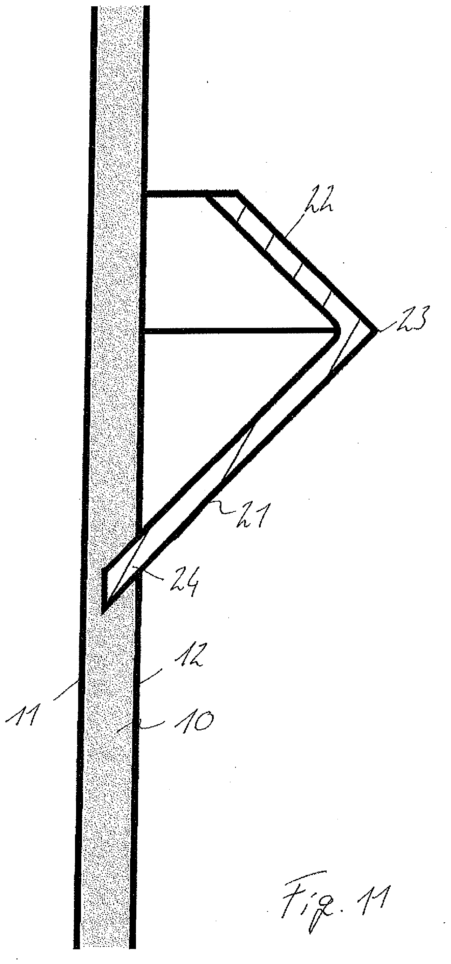

Eine Variante der Erfindung ist in der Figur 11 gezeigt, bei der die Dichtlippe 20, die analog zu der Dichtlippe 20 der Figur 10 ausgebildet ist, in den Grundkörper 10 eingegossen ist. Dazu wird die Dichtlippe 20 zunächst separat hergestellt und dann in dem distalen Bereich des geneigten Abschnitts 21 eingegossen. Der eingegossene Bereich kann eine Verdickung aufweisen, so dass neben einer Vernetzung auch eine formschlüssige Sicherung der Dichtlippe 20 an und in dem Grundkörper 10 erfolgt.

Allen Dichtlippen 20 der Figuren 1 bis 11 ist gemeinsam, dass sie radial umlaufend ausgebildet sind, sich also um den gesamten Umfang des Prothesenliners 1 herum erstrecken. Neben der Ausgestaltung mit nur einer Dichtlippe 20 ist es möglich, mehrere Dichtlippen entlang der Längserstreckung des Prothesenliners 1 vorzusehen.

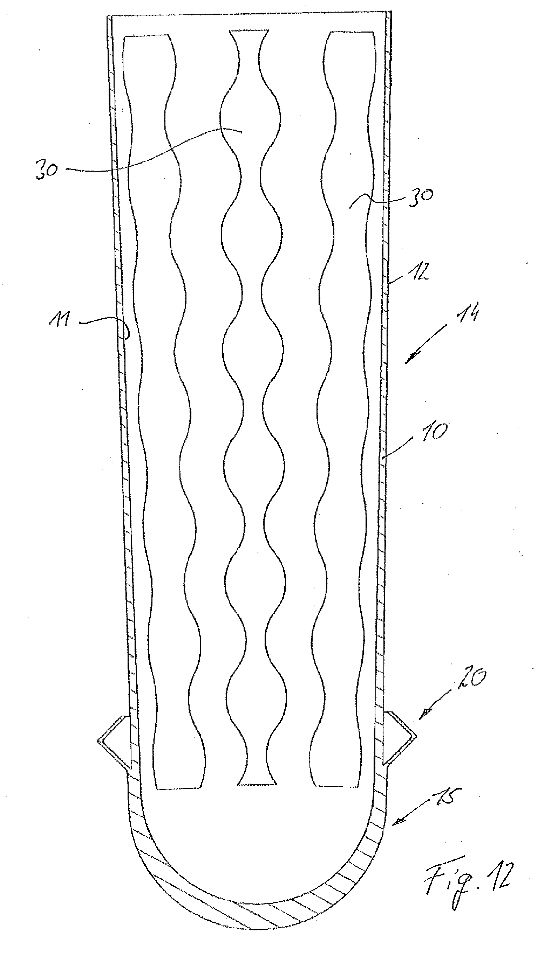

Figur 12 zeigt eine weitere Variante der Erfindung, bei der eine Schnittdarstellung des Prothesenliners 1 mit einer Beschichtung auf der Innenseite 11 des Prothesenliners dargestellt ist. Die Beschichtung ist in Form von Streifen 30 auf der Innenseite 11 aufgebracht, beispielsweise über das CVD-Verfahren, um bereichsweise unterschiedliche Oberflächeneigenschaften realisieren zu können. Neben einer Beschichtung im CVD-Verfahren ist es möglich, in den Bereichen der Streifen 30 andere Materialien einzugießen oder anzugießen oder in einer anderen Art und Weise daran zu befestigen, um die gewünschten Eigenschaften auf der Innenseite 11 des Prothesenliners 1 erzielen zu können. Neben der Beschichtung oder statt der Beschichtung können solcherart geformte Streifen eine Matrix als Stabilisierung oder Versteifung eingegossen werden.

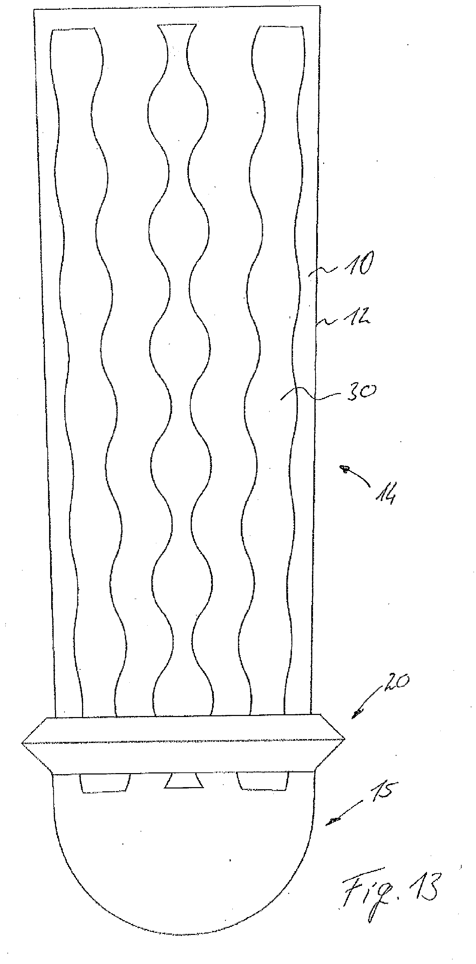

Figur 13 zeigt eine weitere Variante der Erfindung, bei der Streifen 30 auf der Außenseite 12 des Prothesenliners angeordnet oder ausgebildet sind. Die Streifen 30 können geradlinig oder wie dargestellt, in gewellten Formen ausgebildet sein. Die Streifen 30 können gleichmäßig zueinander beabstandet angeordnet sein und beispielsweise aus einem Elastomer bestehen, der von dem Material des Grundkörpers 10 abweicht. Das Elastomer kann beispielsweise als Silikon mit einer höheren Härte als das Silikon des Grundkörpers 10 sein. Die Dichtlippe 20 überdeckt die Streifen 30.

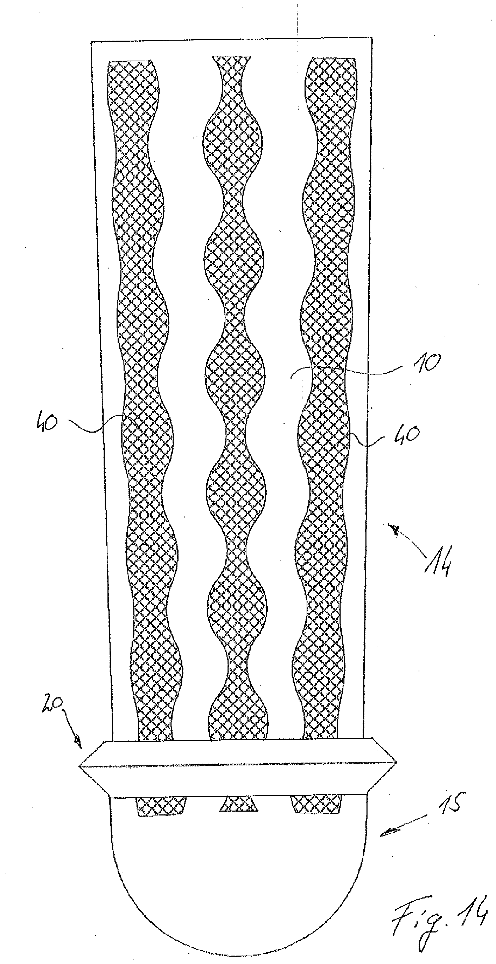

Eine weitere Variante der Erfindung ist in der Figur 14 gezeigt, bei der in dem Grundkörper 10 streifenförmige Matrizes 40 eingebettet sind. Die Matrizes 40 können eine isotrope Elastizität aufweisen und beispielsweise die Dehnung in Längserstreckung des Grundkörpers 10 verhindern. Die Matrizes 40 sind umfänglich zueinander beabstandet angeordnet, so dass zwischen den Matrizes 40 keine unelastische, flexible Matrix angeordnet ist, so dass sich eine Volumenveränderung, insbesondere eine Volumenvergrößerung des Prothesenliners 1 einstellen kann. Durch die Matrizes 40 wird somit die Längselastizität in der Seitenwand 14 eingeschränkt, um den sogenannten Melkeffekt zu verringern, ohne dass die beim Tragen des Prothesenliners 1 auftretende Volumenveränderung zu sehr eingeschränkt wird. Die Matrizes 40 können auch wie in Figur 12 ausgebildet sein, ergänzt um eine zusätzliche elastische Matrix zur Erhöhung des Weiterreißwiderstandes.

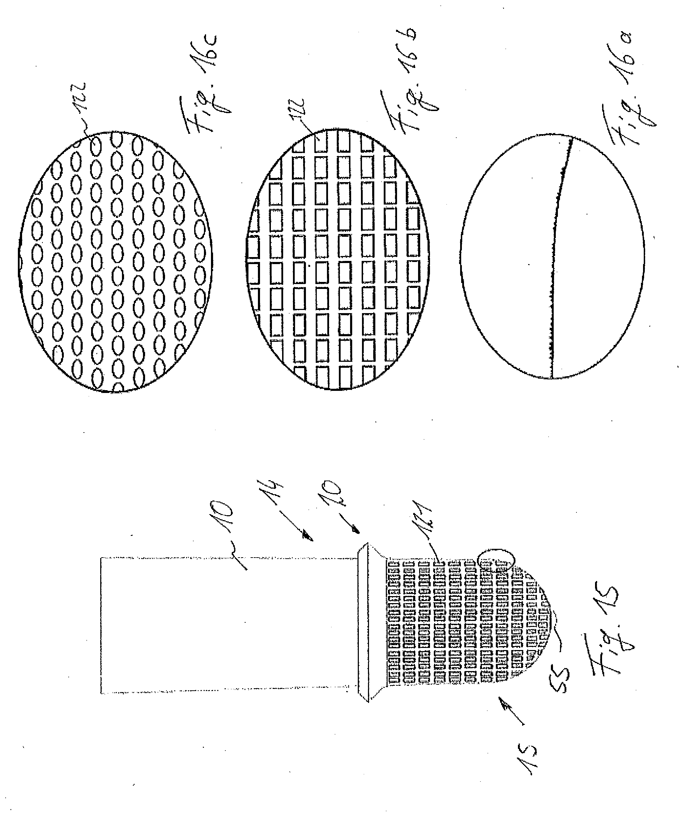

Figur 15 zeigt eine weitere Variante der Erfindung, bei der distal zu der Dichtlippe 20 die Oberfläche auf der Außenseite 12 des Grundkörpers 10 sowohl im Bereich des distalen Endabschnittes 15 als auch in den sich proximal daran anschließenden Bereich an der Seitenwand 14 eine aufgeraute Oberflächenstruktur 121 aufweist.

Die Aufrauhung ist besser in der Figur 16a zu erkennen. Über die Aufrauhung wird verhindert, dass der Bereich distal der Dichtlippe 20 vollflächig an der Innenseite des Prothesenschaftes anliegt und daran anhaftet, so dass kein oder nur ein verminderter Pumpeffekt entsteht. Die Rauheit oder die Oberflächenstruktur auf der Außenseite 12 im Bereich distal der Dichtlippe 20 erleichtert die Ausgestaltung, Verteilung und Ausbildung eines Vakuums distal der Dichtlippe 20.

Die Dichtlippen 20 können auf unterschiedlichen Höhen angeordnet sein. Es können mehrere unterschiedliche Dichtlippenausgestaltungen miteinander kombiniert werden, beispielsweise kann die Ausführungsform gemäß der Figur 3 mit einer oder mehreren Dichtlippenkonfigurationen gemäß der Figuren 2, 6 oder 9 kombiniert werden. Die Dichtlippenkonfiguration gemäß Figur 3 kann als proximale, medial oder distale Dichtlippenkombination mit ein oder mehreren anderen Dichtlippen 20 an der Außenseite 12 des Prothesenliners 1 angeordnet oder ausgebildet sein. Weiterhin können mehrere verschiedene Befestigungsarten der Dichtlippen 20 bei der Anordnung mehrerer Dichtlippen 20 an dem Prothesenliner 1 vorgesehen sein, beispielsweise können Dichtlippen 20 angegossen, eingegossen und/oder anderweitig beispielsweise über das Verkleben oder Verschweißen an dem Grundkörper 10 befestigt oder ausgebildet sein.

Bei dem Einsatz mehrerer Dichtlippen können die Dichtlippen aus unterschiedlichen Materialien mit unterschiedlichen Härten, Elastizitäten oder Oberflächenbeschichtungen ausgebildet sein oder unterschiedliche Beschichtungen aufweisen.

Die unterschiedlichen Möglichkeiten der Kombination der Dichtlippen 20 untereinander können mit einer Innenbeschichtung und/oder Außenbeschichtung des Grundkörpers 10 kombiniert werden. Die Beschichtung kann bereichsweise oder vollflächig auf der Innenseite 11 und/oder Außenseite 12 des Grundkörpers 10 aufgebracht werden.

Zusätzlich zu einer Beschichtung können streifenförmige Materialeinlagerungen aus einem abweichenden Material in dem Grundkörper 10 eingebracht oder auf dem Grundkörper 10 aufgebracht sein. Weiterhin können streifenförmige oder umfänglich geschlossene Matrizes 40 an oder in dem Grundkörper 10 angeordnet sein, insbesondere aufgeklebt, eingebettet oder umhüllt. Auch die Matrizes 40 können in Kombination mit einer Beschichtung auf der Außenseite 12 und/oder Innenseite 11, einer vollflächigen oder teilweisen Beschichtung und/oder einer zusätzlichen Einlagerung oder alternativen Einlagerung von Materialstreifen auf der Innenseite 11 und/oder Außenseite 12 des Grundkörpers 10 kombiniert werden.

In der Figur 16b ist eine weitere Variante der Erfindung dargestellt, bei der regelmäßige Vertiefungen zwischen Erhöhungen 122 angeordnet sind. In der Figur 16b sind die Erhöhungen 122 als Rechtecke oder Blöcke ausgebildet, die zu der Grundfläche des Grundkörpers 10 im distalen Bereich erhaben ausgebildet sind. Zwischen den Erhebungen 122 sind miteinander verbundene Strömungskanäle ausgebildet, so dass eine Oberflächenstruktur oder eine Oberflächenrauigkeit mit erhabenen Noppen ausgebildet wird. Zwischen diesen Noppen kann dann leicht ein Druckausgleich stattfinden, so dass sich bei Anlegen eines Unterdruckes oder eines Vakuums dieser oder dieses gleichmäßig distal von der Dichtlippe 20 aus gesehen verteilt. Dadurch wird verhindert, dass distal zu der Dichtlippe 20 nur bereichsweise ein Unterdruck oder ein ungleichmäßiger Unterdruck entsteht, was zu einem unangenehmen Traggefühl führen kann.

Figur 16c zeigt eine Variante der Erfindung, bei der statt eckiger Erhebungen 122 oder eckiger Blöcke 122 diese oval ausgebildet sind. Die Oberfläche der Erhebungen 122, unabhängig von der Formgebung, ist bevorzugt gradflächig ausgebildet, um eine ausreichende stabile mechanische Anlage an der Innenseite des Prothesenschaftes bewirken zu können, ohne dass es zu lokalen Druckspitzen kommt. Neben einer rechteckigen oder ovalen Formgebung der Erhebungen 122 können diese auch rund, polygonal oder unregelmäßig ausgebildet sein.

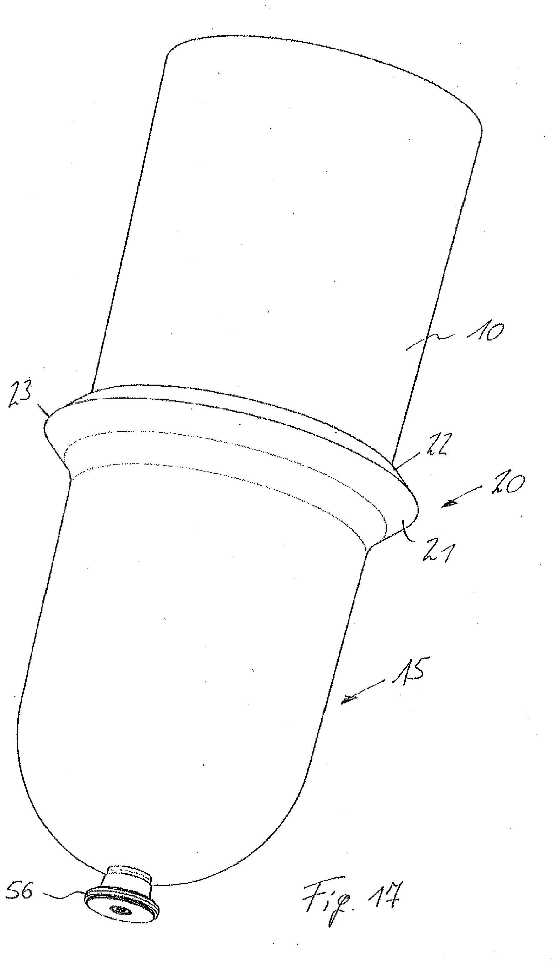

Eine Weiterbildung der Erfindung ist in der Figur 17 gezeigt, bei der an dem distalen Endbereich 15 am distalen Ende des Prothesenliners 1 ein Pumpenkolben 56 angeordnet ist, der mit einer zylindrischen Aufnahme, die nicht dargestellt ist, wechselwirkt. In dem Pumpenkolben 56 ist ein Rückschlagventil angeordnet, das mit einem nicht dargestellten Strömungskanal in dem Kolbenschaft verbunden ist. Über dieses Rückschlagventil wird Luft aus dem nicht dargestellten Zwischenraum zwischen der Außenseite 12 des distalen Endbereiches 15 distal zu der Dichtlippe 20 abtransportiert und über eine Pumpenanordnung in Zusammenwirken mit einem Zylinder in dem Schaft abtransportiert wird.

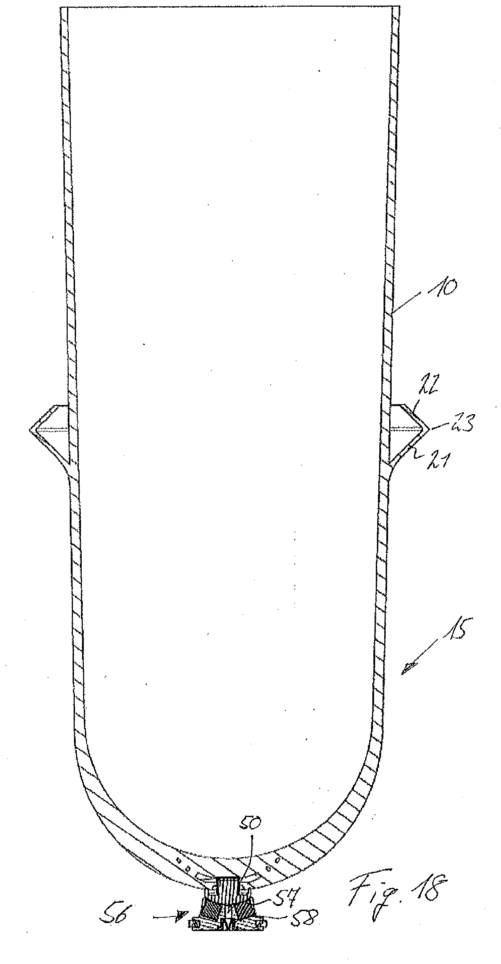

Figur 18 zeigt die Ausgestaltung gemäß Figur 17 in einer Schnittdarstellung. Der Pumpenkolben 56 ist in der Aufnahme 50 im distalen Endbereich des Liners befestigt, insbesondere eingeschraubt. Über den Strömungskanal 57 und das Rückschlagventil 58 wird die Luft aus dem Zwischenraum abtransportiert. Das Rückschlagventil 58 verhindert ein Rückströmen der Luft, so dass sich bei einer Relativbewegung zwischen dem distalen Endabschnitt oder dem Pumpenkolben 56 und dem Prothesenschaft aufgrund der Pumpwirkung ein Unterdruck einstellt. Durch den Unterdruck wird die Dichtkante 23 durch den proximal der Dichtkante 23 befindlichen Überdruck nach distal gedrückt, was zu einer verstärkten Anpressung an die nicht dargestellte Prothesenschaftinnenwand führt.

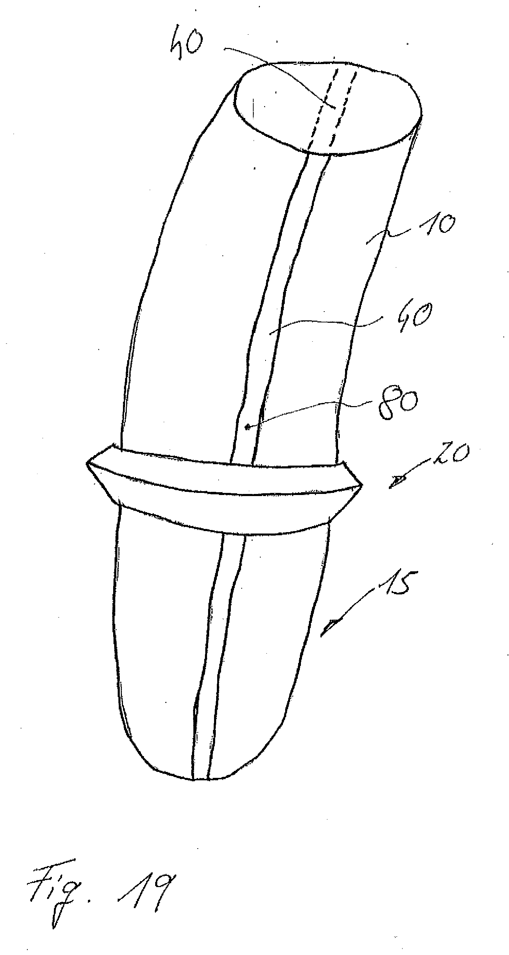

Eine weitere Variante der Erfindung ist in der Figur 19 dargestellt, bei der innerhalb des Grundkörpers 10 oder an der Außenseite 12 oder der Innenseite 11 eine Matrix 40 in Gestalt eines sich in Längserstreckung des Prothesenliners erstreckenden Streifens angeordnet ist. Die Matrizes 40 befinden sich auf im Wesentlichen gegenüberliegenden Seiten des Prothesenliners 1 und verlaufen im angelegten Zustand durch einen Kompromissdrehpunkt 80 eines nicht dargestellten natürlichen Gelenkes, über das der Prothesenliner angelegt wird. Die Matrizes 40 verlaufen ausschließlich medial und lateral zu dem Stumpf und haben den Vorteil, dass durch den medial-lateral-Verlauf eine Gelenkeinbeugung, beispielsweise eine Kniebeugung, nicht oder nur minimal beeinflusst wird. Insbesondere bei flexiblen in elastischen Matrizes 40 wird dadurch eine Längsstabilität des Prothesenliners 1 erreicht, ohne dass die Beweglichkeit des Stumpfes beeinträchtigt wird.

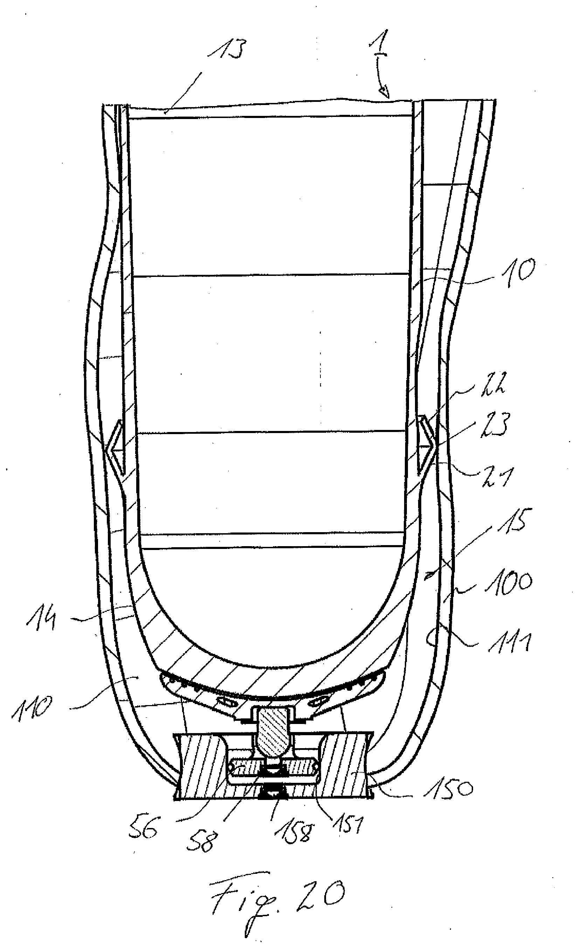

Figur 20 zeigt eine Querschnittansicht durch ein System aus einem Prothesenschaft 100 mit einem Prothesenliner 1, wobei der Prothesenliner 1 eine Variante der Ausgestaltung gemäß der Figuren 17 und 18 aufweist. Die radial äußere Dichtkante 23 liegt an der Innenseite 111 des Prothesenschaftes 100 an. Der Prothesenschaft 100 ist aus einem formstabilen Material ausgebildet und weist einen im wesentlichen geschlossenen Querschnitt auf, zumindest ist der Querschnitt distal zu der Anlagefläche der radial äußeren Dichtkante 23 geschlossen ausgebildet. Zwischen der Außenseite 14 des Prothesenliners 1 und der Innenseite 111 des Prothesenschaftes 100 ist ein Hohlraum 110 ausgebildet, der proximal durch die Dichtkante 23 abgeschlossen wird. Distal und radial wird der Hohlraum 110 durch den Prothesenschaft 100 und auf der Innenseite 111 durch den Prothesenliner 1 abgedichtet. An dem distalen Ende des Prothesenliners 1 ist eine Aufnahmeplatte für den Pumpenkolben 56 angeordnet. Ein Träger 150 mit einem Zylinder 151 ist im distalen Endbereich des Prothesenschaftes 100 ausgebildet. Der Pumpenkolben 56 ist in dem korrespondierend geformten Zylinder 151 eingeführt. In dem Träger 150 ist ein zweites Rückschlagventil 158 angeordnet, so dass Luft aus dem Volumen zwischen dem Kolben 56 und dem Zylinder 151 in die Umgebung herausgedrückt werden kann. Ein Rückströmen von Luft aus der Umgebung ist durch das Rückschlagventil 158 gesperrt. Bei einer Proximalbewegung des Kolbens 56 wird ein relativer Unterdruck distal zu dem Kolben 56 in dem von dem Kolben 56 und dem Zylinder 151 gebildeten Volumen zu dem Hohlraum 110 erzeugt, so dass Luft aus dem Hohlraum 110 in den Zwischenraum durch das Rückschlagventil 58 einströmen kann. Bei einer Distalbewegung des Kolbens 56 aufgrund beispielsweise der Bewegung und beim Auftreten wird diese Luft dann wieder herausgedrückt. Dadurch wird die Festlegung des Prothesenliners 1 in dem Prothesenschaft 100 aufgrund der Vergrößerung des Vakuums verbessert.

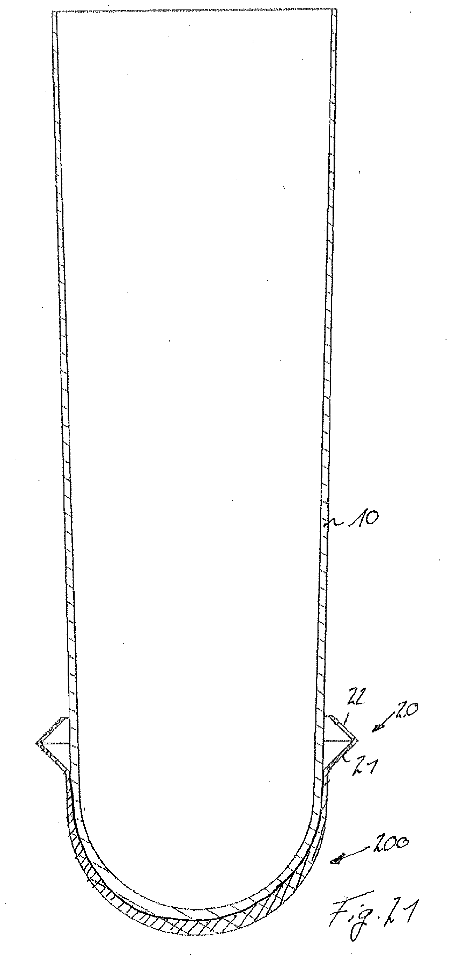

Eine weitere Variante der Erfindung ist in der Figur 21 dargestellt. Der Grundkörper 10 des Prothesenliners 1 besteht dabei zumindest proximal der Dichtlippe 20 aus einem atmungsaktiven Material, beispielsweise aus einem Textil, insbesondere einem 3D-Abstandsgewirk, das als geschlossener, einseitig offener Liner ausgebildet und den Stumpf vollumfänglich umfasst. Der Liner und der Grundkörper 10 weisen einen geschlossenen, distalen Endbereich 15 auf, der den nicht dargestellten Stumpf distal und umfänglich umgibt. An der Außenseite des Grundkörpers 10 ist eine abdichtende Kappe 200 angeformt und angegossen, die aus einem elastomeren, luftundurchlässigen oder im Wesentlichen luftundurchlässigen Material besteht, beispielsweise aus Silikon oder einem Polymer. Die Dichtkappe 200 bildet die Dichtlippe 20 an ihrem proximalen Endabschnitt aus, wodurch eine Abdichtung distal zu der Dichtlippe 20 erreicht wird. Die Dichtkappe 200 kann, wie in der Figur 21 gezeigt, relativ weit distal enden, alternativ kann die Dichtkappe 200 sich bis zur Mitte des Grundkörpers 10 oder proximal darüber hinaus erstrecken. Dann befindet sich ein luftdurchlässiger, beispielsweise textiler Grundkörper 10 jeweils auf der Innenseite der Dichtkappe 200 und wird über die Dichtlippe 20 gegenüber dem Prothesenschaft abgedichtet. Die Dichtkappe 200 ist dabei großflächig mit der Außenseite 14 des Grundkörpers 10 verbunden, beispielsweise durch Vergießen oder Angießen der Dichtkappe 200 an den Grundkörper 10 oder durch Ankleben oder andere Befestigungsarten. Die Dichtkappe 200 kann über den Grundkörper 10 gezogen werden, eine dauerhafte Befestigung kann entfallen, wenn die Haftung an der Außenseite des Grundkörpers 10 groß genug ist.

Alternativ zu einer großflächig umfänglichen Befestigung des Grundkörpers 10 an der Dichtkappe 200 ist es möglich, den Grundkörper 10 als einen Schlauch aus einem luftdurchlässigen, insbesondere textilen Material auszugestalten, an dessen distalen Ende die Dichtkappe 200 mit der Dichtlippe 20 angegossen wird. Mit beiden Varianten wird das Ziel erreicht, einen atmungsaktiven Liner bereitzustellen, über den eine distale Verriegelung in einem Prothesenschaft bei gleichzeitiger Volumenkontrolle erreicht werden kann. Über die Ausgestaltung des Grundkörpers 10 aus einem atmungsaktiven, luftdurchlässigen Material kann auch eine Schweißabfuhr gewährleistet sein. Neben einer Ausgestaltung des atmungsaktiven, luftdurchlässigen Materials aus einem 3D-Abstandsgewirk können andere Textilien oder offenporige Materialien wie gelochte Elastomere, Schäume oder dergleichen zum Einsatz kommen.

Die Innenseite 11 des Liners 1, unabhängig aus welchem Material der Grundkörper 10 besteht, kann mit einer Beschichtung versehen sein, die neben einem Dichteffekt ein Anhaften auf der Haut der Nutzers ermöglicht oder verbessert. Alternativ oder ergänzend kann die Innenseite 11 des Liners 1 mit einer Oberflächenstruktur oder einer Beschichtung versehen sein, die einen richtungsabhängigen Widerstand bereitstellt. Der Widerstand ist gegenüber einer Auszugsbewegung oder einer Drehbewegung um die Längsachse des Stumpfes erhöht, so dass ein Einsteigen in den Liner erleichtert und ein Herausziehen erschwert wird. Zudem wird die Position des Liners an dem Stumpf gesichert. Es wird ein sogenannter "dog skin"-Effekt bereitgestellt, ähnlich einem Strichvelours.

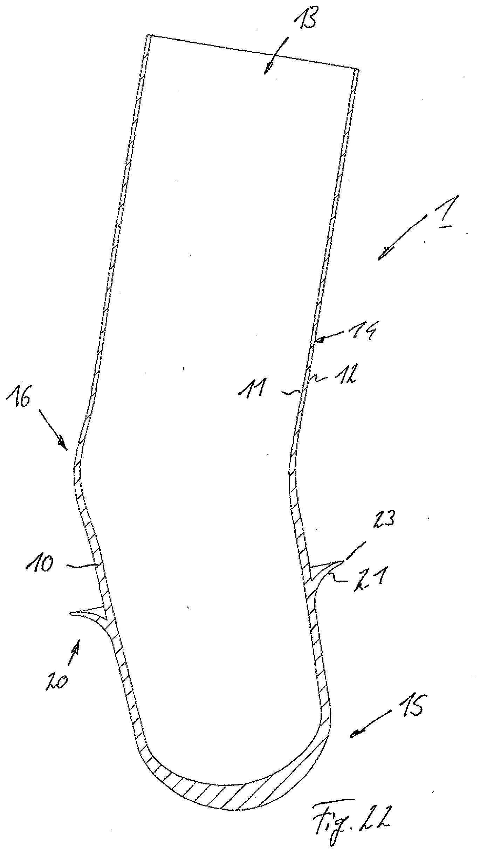

In der Figur 22 ist in einer Schnittdarstellung ein Prothesenliner 1 gezeigt, der in seinem Aufbau grundsätzlich dem Liner gemäß Figur 3 entspricht. Auch hier ist in dem distalen Endbereich 15 eine vergrößerte Materialstärke verglichen mit der Materialstärke in der Seitenwand 14 vorgesehen. Die Dichtlippe 20 weist einen gekrümmten Querschnitt auf und ist einstückig zusammen mit dem Grundkörper 10 ausgebildet. Der geneigte Abschnitt 21 wölbt sich in einer Biegung nach außen, so dass sich auf der distalen Seite der Dichtkante 23 eine konkave Wölbung ergibt, wenn man von schräg unten auf die Dichtlippe 20 schaut. Auf der Rückseite, also der proximalen Seite der Dichtlippe 20, ergibt sich eine korrespondierende Wölbung, die aus gleicher Richtung entsprechend konvex ausgebildet ist, so dass sich ein V-förmiger Zwischenraum zwischen der proximalen Seite der Dichtlippe 20 und der Außenwand 12 der Seitenwand 14 des Prothesenliners 1 einstellt. Weiter proximal zu der Dichtlippe 20 ist die Seitenwand 14 vorflektiert, so dass der Prothesenliner 1 in dem nicht angelegten, unbelasteten Zustand bei einer angenommenen Formstabilität ohne von außen einwirkenden Kräften eine Beugung oder einen Knick aufweist, der so ausgebildet ist, dass in diesem Bereich ein natürliches Gelenk aufgenommen werden kann. Bei einer Ausgestaltung des Prothesenliners 1 als Liner für eine untere Extremität kann bis in den distalen Endbereich 15 ein Unterschenkelstumpf eingeführt werden. Der Prothesenliner 1 ist so bemessen, dass die Krümmung in dem Bereich des natürlichen Kniegelenkes angeordnet ist. In einem frontalen Bereich ist eine Auswölbung 16 für eine Kniescheibe ausgebildet, um eine verbesserte Anlage an die untere Extremität zu gewährleisten. Bei einer Ausgestaltung als Liner für eine obere Extremität ist die Auswölbung 16 auf der Rückseite im Bereich eines Ellenbogens angeordnet, während die gegenüberliegende Einbeugung im Bereich der Ellenbeuge angeordnet ist. Proximal zu der Auswölbung 16 verjüngt sich der Grundkörper 10 weiter, so dass insgesamt drei Materialstärken mit jeweils dazwischen liegenden sanften Übergängen vorhanden sind. Die unterschiedlichen Materialstärken sehen in dem distalen Endbereich 15 eine vergrößerte Materialstärke zur Aufnahme von Druckkräften und zu deren gleichmäßiger Verteilung vor. Daran anschließend ist eine mittlere Materialstärke vorhanden, um eine ausreichende Stabilität und ausreichend Material für die Anformung einer Dichtlippe 20 bereitzustellen. Eine dünne Materialstärke befindet sich ab der Auswölbung 16 in proximaler Richtung, um ein angenehmes Traggefühl im Bereich des Oberarmes oder Oberschenkels bei einem angelegten Prothesenliner 1 zu erreichen.

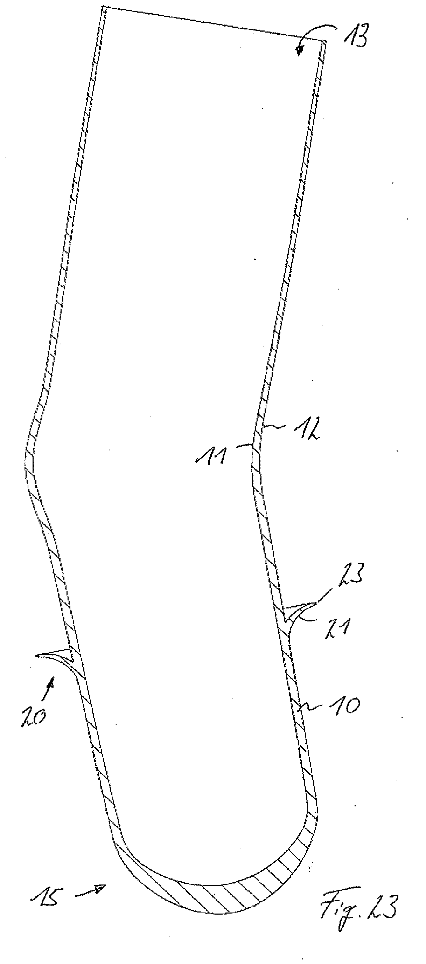

Figur 23 zeigt eine weitere Variante des Liners und eine Weiterbildung des Liners gemäß Figur 3. Der Liner gemäß Figur 23 weist einen längeren Einführbereich ab der Auswölbung 16 auf, ebenfalls ist die Dichtlippe 20 weiter proximal von dem distalen Endbereich 15 angeordnet, so dass der Liner gemäß Figur 23 insbesondere für Patienten mit einem längeren Stumpf distal zu einem natürlichen Gelenk geeignet ist.

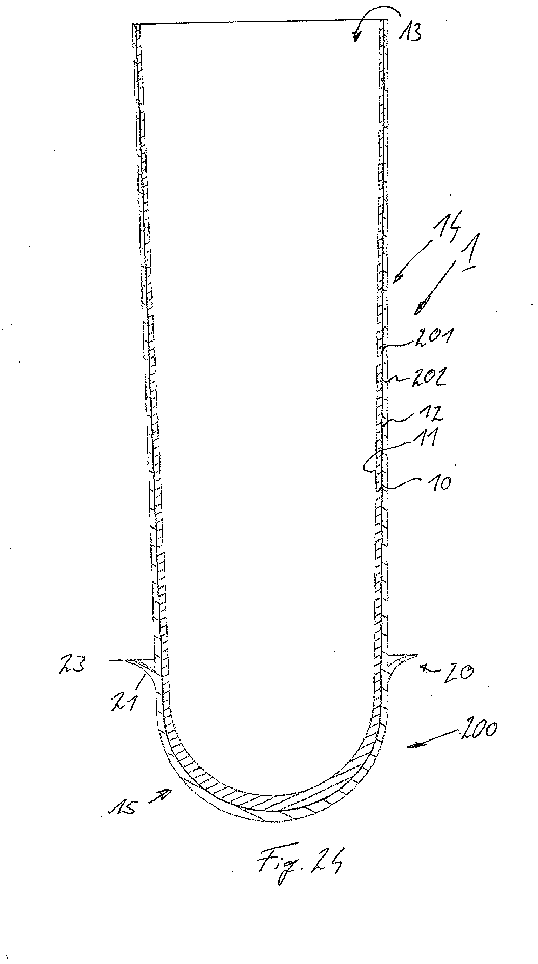

In der Figur 24 ist eine weitere Variante des Prothesenliners 1 in Querschnittsansicht gezeigt. Die Dichtlippe 20 weist dabei die Form der Dichtlippe 20 gemäß der Figuren 22 und 23 auf und ist also entsprechend der Dichtlippe 20 in Figur 3 ausgebildet. Im Unterschied zu der Figur 3 ist die Dichtlippe 20 wesentlich näher an dem distalen Endbereich 15 des Prothesenliners 1 angeordnet. Auch hier ist im distalen Endbereich 15 eine leichte Materialverdickung vorgesehen. Die Dichtlippe 20 ist einstückig mit dem übrigen äußeren Material ausgebildet. Die Dichtlippe 20 ist ein Teil einer Dichtkappe 200, die sich in dem Ausführungsbeispiel der Figur 24 über die gesamte Länge der Seitenwand 14 des Prothesenliners 1 erstreckt. Die Dichtkappe 200 weist eine Innenseite 201 und eine nach außen gerichtete Außenseite 202 auf, wobei sich von der Außenseite 202 die Dichtlippe 20 weiter radial nach außen erstreckt. An der Innenseite 201 der Dichtkappe 200 ist der Grundkörper 10 angeordnet. Die Außenseite 12 des Grundkörpers 10 liegt an der Innenseite 201 der Dichtkappe 200 an und ist mit dieser gekoppelt, beispielsweise über haftende Beschichtungen an der Außenseite 12 bzw. Innenseite 201, eine Verklebung, eine Verschweißung oder über kleine Formschlusselemente, die an der Außenseite 12 und Innenseite 201 korrespondierend zueinander ausgebildet sind. Durch korrespondierend ausgebildete Vorsprünge und Ausnehmungen oder Hakenbereiche kann neben einer stoffschlüssigen Verbindung von Grundkörper 10 und Dichtkappe 200 eine formschlüssige Verbindung hergestellt werden. Der Vorteil einer formschlüssigen Verbindung besteht darin, dass der Grundkörper 10 von der Dichtkappe 200 wieder getrennt werden kann. Der Grundkörper 10 weist ebenfalls in dem distalen Endbereich 15 eine erhöhte Materialstärke im Vergleich zu dem proximalen Endbereich auf. Die Materialstärke kann sich kontinuierlich von dem distalen Endbereich 15 bis zu der Einstiegsöffnung 13 verringern. Es ist auch möglich, Materialabstufungen vorzusehen, wobei die Innenseite 11 des Grundkörpers 10 bevorzugt glattflächig und ohne Absätze ausgebildet ist. Die Abstufung erfolgt an der Außenseite 12 des Grundkörpers 10 und kann mit Querschnittsvergrößerungen oder Verringerungen der Dichtkappe 200 einhergehen, so dass dort eine formschlüssige Verriegelung entgegen einer Auszugsrichtung von dem distalen Endbereich 15 in Richtung auf die proximale Einstiegsöffnung 13 gegeben ist.

Die Formgebung des Prothesenliners 1 gemäß Figur 24 ist geradlinig, hülsenförmig und mit einem geschlossenen Querschnitt. Grundsätzlich ist es auch möglich, eine Vorflektion, wie in den Figuren 22 und 23 gezeigt, bei einem solchen mehrteiligen oder aus zwei unterschiedlichen Materialien hergestellten Prothesenliner 1 gemäß Figur 24 vorzusehen. Der Grundkörper 10 kann aus einem atmungsaktiven Material oder einem 3D-Abstandsgewirk hergestellt sein, die Innenseite 11 kann vollständig oder teilweise mit einer Haftbeschichtung versehen sein, um ein Anhaften des Prothesenliners 1 an dem Stumpf zu ermöglichen. Grundsätzlich ist es auch möglich, dass der Grundkörper 10 aus einem Elastomer hergestellt ist. Die Dichtkappe 200 kann aus einem von dem Grundkörper 10 abweichenden Material hergestellt sein, wie im Zusammenhang mit der Figur 21 beschrieben.

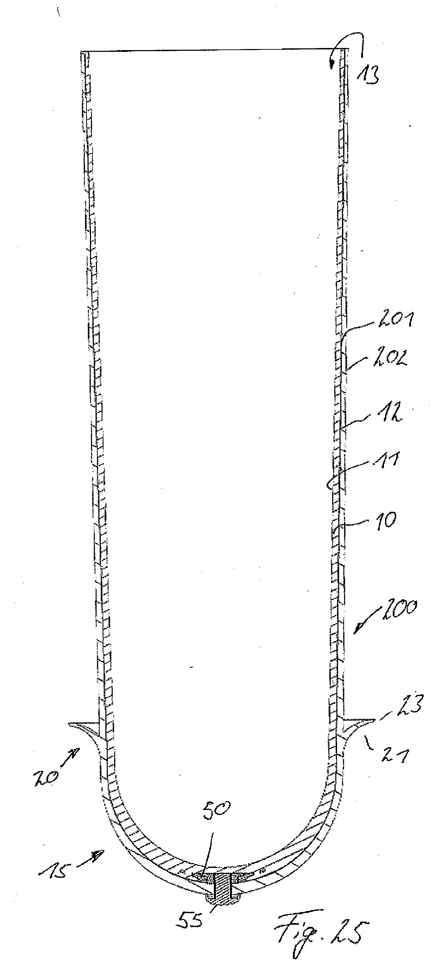

Figur 25 zeigt eine Variante der Figur 24, bei der zusätzlich zu dem Aufbau des Prothesenliners 1 im distalen Endbereich 15 ein Teil einer Pumpanordnung oder eines Befestigungselementes dargestellt ist, ähnlich der Figur 7. Innerhalb einer Aufnahme 50, die in dem Grundkörper 10 eingebettet ist, ist ein Pin 55 eingesetzt, insbesondere eingeschraubt, über den eine Verbindung zu einem Shuttle Lock oder einer Pumpanordnung, wie sie in der Figur 20 gezeigt ist, hergestellt werden kann. An den Pin 55 kann sich beispielsweise ein Pneumatikkolben anschließen, der in einem Zylinder geführt ist und bei Belastung und Benutzung des Prothesenliners eine Pumpbewegung innerhalb des Zylinders ausführt.

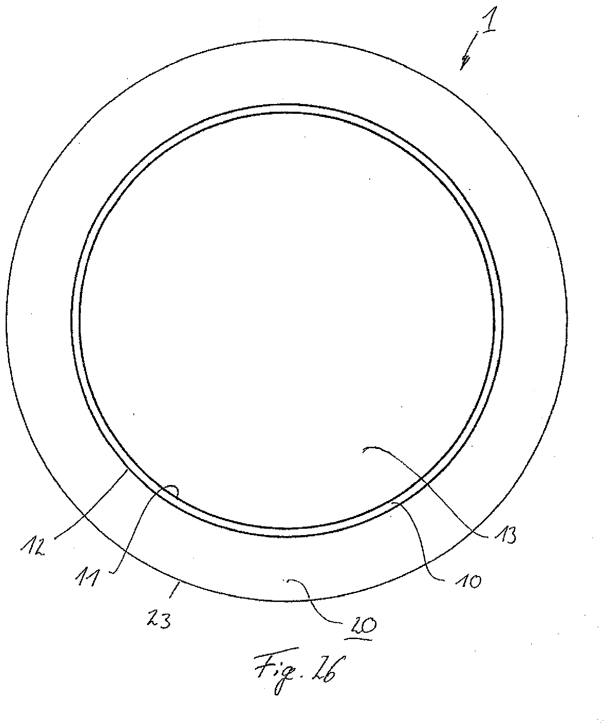

Figur 26 zeigt eine Draufsicht auf einen Prothesenliner 1 im unbelasteten Zustand. Der Prothesenliner 1 weist dabei einen im Wesentlichen kreisförmigen Querschnitt des Grundkörpers 10 auf, um den herum sich die ebenfalls kreisförmige Dichtlippe 20 radial nach außen erstreckt. Die Dichtlippe 20 weist einen kreisförmigen Querschnitt auf, wobei die Dichtlippe 20 und der Grundkörper 10 konzentrisch zueinander angeordnet sind, so dass die Dichtlippe 20 eine gleichbleibende radiale Erstreckung oder Breite aufweist. Der Außenumfang der Dichtkante 23 ist kreisförmig, ebenso wie die Außenseite 12 und die Innenseite 11 des Grundkörpers 10.

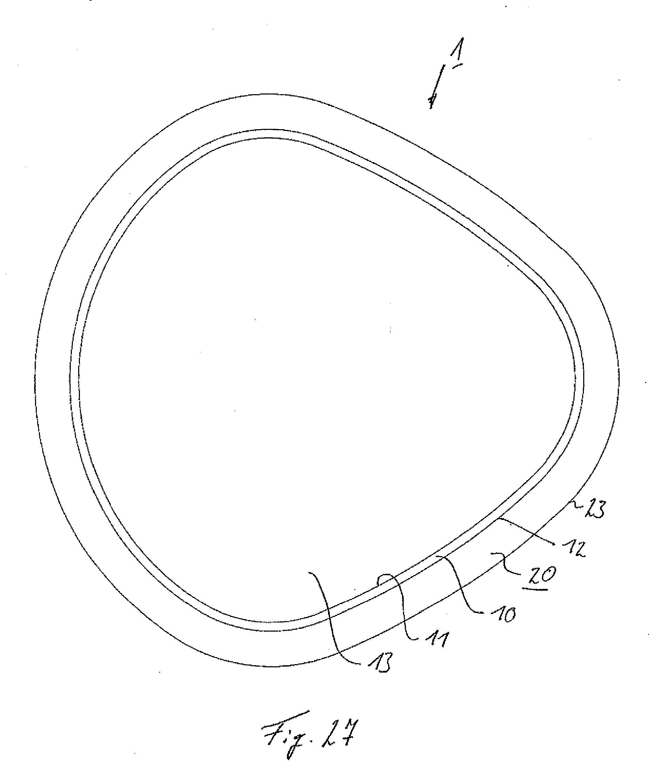

Eine Variante der Formgebung des Prothesenliners 1 ist in der Figur 27 dargestellt, die ebenfalls eine Draufsicht auf einen unbelasteten Prothesenliner 1 darstellt. Der Prothesenliner 1 hat somit die Form, die er am Ende des Formgebungsprozesses aufweist, ohne dass Seitenkräfte oder Querkräfte auf die Seitenwand 14 wirken. Statt eines kreisförmigen Umfanges sowohl der Dichtkante 23 als auch der Außenseite 12 bzw. Innenseite 11 des Grundkörpers 10 weist der Prothesenliner 1 eine angenährt dreieckige Form auf, wobei die Ecken abgerundet sind und die Seiten des Dreiecks ebenfalls eine Abrundung nach außen aufweisen. Alle Prothesenliner gemäß der vorstehenden Figuren 1 bis 25 können sowohl eine kreisförmige Grundform gemäß Figur 26 oder aber eine davon abweichende, polygonale oder angenähert polygonale oder insbesondere dreieckige Ausgestaltung gemäß Figur 27 aufweisen. Ebenfalls kann eine Vorflektion oder Vorwölbung gemäß der Figuren 22 und 23 auch mit einem Querschnitt abweichend von einem kreisförmigen Querschnitt ausgebildet werden.

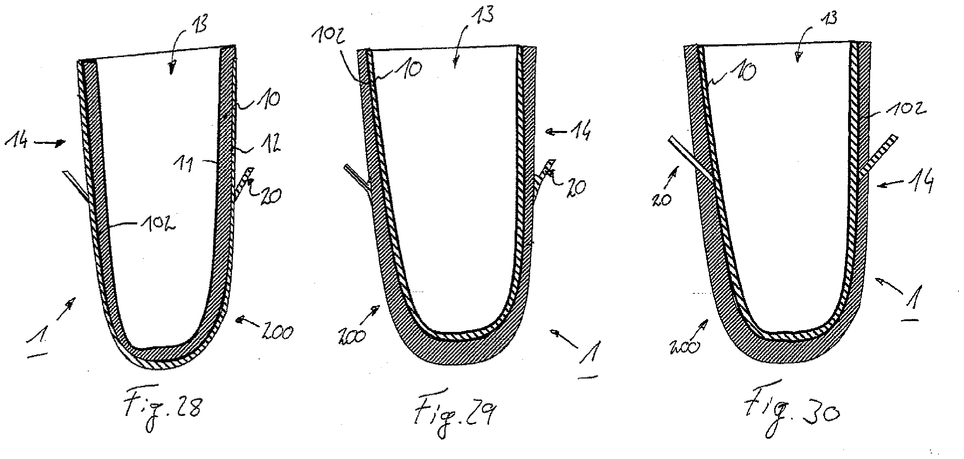

In der Figur 28 ist in einem Längsschnitt eine weitere Variante der Erfindung dargestellt, bei der der Prothesenliner 1 mehrschichtig aufgebaut ist. Der Grundkörper 10 weist an seiner Außenseite 12 eine Dichtkappe 200 auf, die sich über die gesamte Außenseite 12 des Grundkörpers 10 erstreckt. Der Grundkörper 10 ist in dem dargestellten Ausführungsbeispiel aus einem thermoplastischen Polymer (TPE), insbesondere einem thermoplastischen Copolymer ausgebildet, da aufgrund des in dem TPE enthaltenen Weißöls dieses Material hautpflegende Eigenschaften aufweist. Die Dichtkappe 200 kann zunächst glattwandig ausgebildet und mit einer im Wesentlichen gleichmäßigen Wandstärke auf dem Grundkörper 10 aufgebracht sein und aus einem Silikon bestehen. Zur Verbesserung der Anhaftung des Silikons der Dichtkappe 200 an dem Grundkörper 10 kann eine Zwischenschicht 102 in Gestalt einer textilen Zwischenlage oder einer CVD-Beschichtung, bevorzugt aus Parylen, aufgebracht sein. Die Dichtlippe 20 kann angegossen, aufgeklebt oder als Einleger in der Dichtkappe 200 eingearbeitet sein, alternativ kann die Dichtlippe 20 auch integral in der Dichtkappe 200 angeformt sein.

Eine alternative Ausgestaltung der Figur 28 sieht vor, dass statt des Grundkörpers 10 aus einem TPE dieser aus einem Polyurethan besteht.

Figur 29 zeigt eine weitere Variante der Erfindung mit einem Prothesenliner 1, der einen Grundkörper 10 aufweist, auf dessen Außenseite 12 eine Dichtkappe 200 angeordnet ist, die sich über die gesamte Länge der Seitenwand 14 bis zu der Einführöffnung 13 oder bis nahezu zum proximalen Rand der Einführöffnung 13 erstreckt. Der Grundkörper 10 besteht vorteilhafterweise aus einem Silikon. Silikon hat den Vorteil, dass es leicht zu reinigen ist, so dass Verschmutzungen auf der Innenseite 11 des Grundkörpers 10 leicht zu entfernen sind. Die Dichtkappe 200 kann aus einem von Silikon abweichenden Material hergestellt sein, beispielsweise aus einem Polyurethan oder einem Copolymer, insbesondere einem TPE. Die an der Außenseite der Dichtkappe 200 angeformte oder ausgebildete Dichtlippe 20 kann aus dem gleichen Material wie die Dichtkappe 200 bestehen. Alternativ zu einer Ausgestaltung der Dichtlippe 20 aus dem Material der Dichtkappe 200 oder aus einem gleichartigen Material besteht die Möglichkeit, die Dichtlippe 20 aus einem Silikon herzustellen, so dass sich ein Aufbau aus einem Grundkörper 10 aus Silikon, einer Dichtkappe 200 aus einem Polyurethan oder einem Copolymer, insbesondere einem TPE und einer daran angeformten Dichtlippe 20 wiederum aus einem Silikon ergibt.

Um die Dichtkappe 200 oder Zwischenlage an dem Grundkörper 10 aus Silikon sicher und einfach festlegen zu können, kann eine Zwischenschicht 102 zwischen dem Silikon des Grundkörpers 10 und der Dichtkappe 200 oder dem Außenliner aus einem Polyurethan oder einem TPE aufgebracht sein. Die Zwischenschicht 102 kann durch ein CVD-Verfahren als ein Haftvermittler aufgebracht werden, beispielsweise eine Schicht aus Parylen. Alternativ kann zur Verbesserung der Anhaftung des Polyurethans oder des TPE die Zwischenschicht 102 als eine textile Zwischenlage ausgebildet sein. An der Außenseite der Dichtkappe 200 ist dann in dem Bereich der Befestigung der separat gefertigten Dichtlippe 20 ebenfalls eine haftvermittelnde Schicht, beispielsweise aus Parylen im CVD-Verfahren aufgebracht oder bereichsweise eine textile Außenschicht angeordnet, die dann zumindest teilweise durch die angeformte, aufgegossene oder aufgeklebte Dichtlippe 20 überdeckt wird.

Eine weitere Variante der Erfindung ist in der Figur 30 dargestellt, bei der der Grundkörper 10 aus einem Silikon hergestellt ist, wobei die Dichtlippe 20 ein integraler Bestandteil des Grundkörpers 10 ist oder an dem Grundkörper 10 als separates Bauteil befestigt ist. Die Zwischenschicht 102 zur verbesserten Anhaftung der Dichtkappe 200 an der Außenseite des Grundkörpers 10 kann aus Parylen oder einem anderen, die Haftung der Dichtkappe 200 an dem Silikon des Grundkörpers 10 verbessernden Material ausgebildet sein. Die Zwischenschicht 102 wird bevorzugt über ein CVD-Verfahren aufgebracht. Eine textile Zwischenschicht als Zwischenlage 102 kann eingegossen werden. Bevorzugt besteht die Dichtkappe 200 oder der Außenliner aus einem Polyurethan, der ebenfalls an seiner Außenseite mit einer Beschichtung versehen sein kann, die mit dem CVD-Verfahren aufgebracht sein kann.

Bei den sogenannten Hybridlinern, die aus mehreren Materialien hergestellt worden sind, ergibt sich der Vorteil, dass unterschiedliche Materialeigenschaften an den jeweiligen Positionen unterschiedliche Vorteile verwirklichen. Silikon ist leicht zu reinigen und bildet einen besonders haltbaren Liner oder eine Linerschicht aus. Polyurethane oder thermoplastische Elastomere, insbesondere Copolymere, bilden eine einstellbare Funktionsschicht aus, so dass insgesamt ein Prothesenliner 1 erreicht werden kann, der die Bedürfnisse und Anforderungen des Prothesennutzers noch besser erfüllt als ein Prothesenliner, der aus nur einem Material besteht. Bessere Eigenschaften können bei gleicher Materialdicke erreicht werden bzw. bei einer geringeren Materialstärke können vergleichbare Eigenschaften erreicht werden. Insbesondere kann die Silikonschicht dünner im Vergleich zu einem Prothesenliner 1 aus nur einem Silikon hergestellt werden.

Statt die Außenseite 202 der Dichtkappe 200 mittels CVD-Verfahren zu beschichten, kann auch ein Textil oder eine textile Außenschicht daran angeordnet sein, die von der Dichtlippe 20 durchragt wird oder auf der die Dichtlippe 20 angeordnet ist.

Die Oberfläche der Innenseite 11 des Prothesenliners 1 kann bei allen Ausführungsformen des Prothesenliners aufgeraut sein. Die aufgeraute Struktur bewirkt eine Oberflächenvergrößerung, über die eine bessere Verbindung der Innenseite 11 des Prothesenliners 1 auf der Haut des Stumpfes bewirkt werden kann. Aufgrund der verbesserten Anhaftung des Prothesenliners 1 an dem Stumpf erfolgt darüber eine verbesserte Kopplung an eine Prothese, insbesondere einem Prothesenschaft, wodurch die Handhabung der gesamten Prothese für den Patienten verbessert wird. Darüber hinaus führt die aufgeraute Oberfläche zu einem angenehmen Tragegefühl und einer angenehmen Haptik. Die aufgeraute Oberflächenstruktur wird dadurch erreicht, dass der Formkern, auf dem der Grundkörper 10 des Liners 1 gefertigt wird, eine aufgeraute Oberfläche aufweist. Eine aufgeraute Oberfläche kann durch das Aufbringen oder Einbringen eines regelmäßigen Musters aus Erhebungen und Vertiefungen erreicht werden. Ebenfalls können unregelmäßige Erhebungen und Vertiefungen in der Oberfläche des Formkerns aufgebracht oder eingebracht sein, um eine entsprechende Oberflächenstruktur auf der Innenseite 11 des Grundkörpers 10 zu erzielen.

Um den Prothesenliner 1 in einer vorflektierten Form zu erhalten, wie er beispielsweise in den Figuren 22 und 23 gezeigt ist, kann der Grundkörper 10 aus einem TPE oder mit einer Schicht aus TPE auf ein flektiertes Stumpfmodell gezogen werden. Nach dem Aufbringen auf ein Stumpfmodel wird der Liner 1 getempert, so dass die Form des Stumpfmodels angenommen wird. Die Temperaturen zum Tempern sind abhängig von der chemischen Zusammensetzung des TPE und liegen üblicherweise zwischen 60°C und 70°C.

Insbesondere bei vorflektierten Linern kann in dem Bereich der Kniekehle oder der Beugung, die der Auswölbung 16 gegenüberliegt, ein dünneres Material als auf der gegenüberliegenden Seite angeordnet sein, um eine Einbeugung des Prothesenliners 1 keinen zu großen Widerstand entgegenzusetzen und einer Faltenbildung entgegenzuwirken bzw. eine Faltenbildung zu reduzieren.

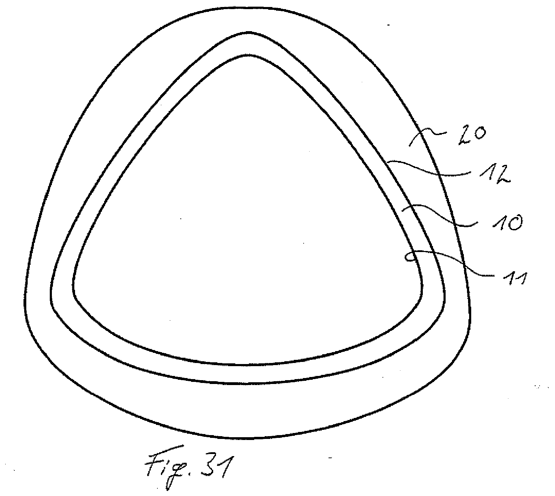

Figur 31 zeigt einen Horizontalschnitt, bei dem die angenähert dreieckige Grundform des Prothesenliners 1 zu erkennen ist. Eine solche Linerform, wie sie ähnlich auch in der Figur 27 gezeigt ist, ist insbesondere für die Anwendung bei einem Unterschenkelliner vorteilhaft, da ein Schienbein mit dem dazugehörigen Wadenmuskel eine angenähert dreieckige Form im Querschnitt ausbildet.

Um bei einer separat angebrachten Dichtlippe 20 bei einer dreieckigen Grundform des Grundkörpers 10 die Dichtlippe 20 leicht angießen oder ankleben zu können, kann der Grundkörper 10 aufgeblasen oder auf einen Träger mit einem runden Querschnitt aufgebracht werden. Nach dem Aufbringen des Grundkörpers 10 auf einen solchen Träger wird dann die Dichtlippe 20 angegossen oder angeklebt und der Liner nach dem Festlegen oder Antrocknen von dem Träger entfernt. Auf diese Art und Weise kann der Grundkörper 10 eine nahezu dreieckige Querschnittsform aufweisen, während die Dichtlippe 20 in der Draufsicht eine angenähert runde Kontur aufweist.

## Patentansprüche

1. Prothesenliner, der zum Anlegen über einen Stumpf einer Gliedmaße vorgesehen ist, mit einem Grundkörper (10), der eine zur Haut des Stumpfes hin gerichtete Innenseite (11) und eine von dem Stumpf weg gerichtete Außenseite (12) aufweist, mit einer proximalen Einstiegsöffnung (13) und einer Seitenwand (14), die sich von der Einstiegsöffnung (13) bis zu einem geschlossenen distalen Endabschnitt (15) erstreckt, wobei an der Außenseite (12) des Grundkörpers (10) zumindest eine Dichtlippe (20) angeordnet ist, die von dem Grundkörper (10) radial nach außen ragt, **dadurch gekennzeichnet, dass** der Umfang der Seitenwand (14) im unbelasteten Zustand eine angenähert dreieckige Form mit abgerundeten Ecken und die Dichtlippe (20) im unbelasteten Zustand eine angenähert dreieckige Form mit abgerundeten Ecken oder eine angenähert runde Kontur aufweist.

2. Prothesenliner nach Anspruch 1, **dadurch gekennzeichnet, dass** die Dichtlippe (20) einen in Richtung auf die Einstiegsöffnung (13) geneigten Abschnitt (21) aufweist.

3. Prothesenliner nach Anspruch 2, **dadurch gekennzeichnet, dass** sich an den geneigten Abschnitt (21) ein in Richtung auf den Grundkörper gerichteter Proximalabschnitt (22) anschließt.

4. Prothesenliner nach einem der voranstehenden Ansprüche, **dadurch gekennzeichnet, dass** die Dichtlippe (20) an dem Grundkörper (10) angegossen ist.

5. Prothesenliner nach einem der voranstehenden Ansprüche, **dadurch gekennzeichnet, dass** die Dichtlippe (20) als separates Bauteil ausgebildet und an dem Grundkörper (10) befestigt oder angegossen ist.

6. Prothesenliner nach einem der voranstehenden Ansprüche, **dadurch gekennzeichnet, dass** die Außenseite (12) textillos ausgebildet oder mit einer reibungsverringernden Beschichtung versehen ist.

7. Prothesenliner nach einem der voranstehenden Ansprüche, **dadurch gekennzeichnet, dass** die Außenseite (12) zumindest teilweise mit einer aufgerauten Oberflächenstruktur (121), insbesondere distal zu der Dichtlippe (20), versehen ist, die eine Vakuumverteilung begünstigt.

8. Prothesenliner nach einem der voranstehenden Ansprüche, **dadurch gekennzeichnet, dass** an der Innenseite (11) und/oder Außenseite (12) Streifen (30) aus einem von dem Material des Grundkörpers (10) abweichenden Material angeordnet sind.

9. Prothesenliner nach einem der voranstehenden Ansprüche, **dadurch gekennzeichnet, dass** in dem Grundkörper (10) zumindest eine Matrix (40) mit isotropen Elastizitäten eingebettet ist.

10. Prothesenliner nach Anspruch 9, **dadurch gekennzeichnet, dass** mehrere Matrizes (40) mit unterschiedlichen Elastizitäten in dem Grundkörper eingebettet sind.

11. Prothesenliner nach Anspruch 9 oder 10, **dadurch gekennzeichnet, dass** zumindest eine erste unelastische Matrix (40) in Umfangsrichtung beabstandet zu einer elastischen zweiten Matrix (40) in dem Grundkörper (10) eingebettet ist.

12. Prothesenliner nach zumindest einem der Ansprüche 9 bis 11, **dadurch gekennzeichnet, dass** das die Matrix (40) medial und lateral angeordnet ist, insbesondere im Bereich eines Kompromissdrehpunktes (80) eines Gelenkes.

13. Prothesenliner nach einem der voranstehenden Ansprüche, **dadurch gekennzeichnet, dass** an dem Grundkörper (10) eine Kolbenpumpe, ein Pumpenkolben (56) oder in dem Grundkörper (10) eine Aufnahme (50) für eine Pumpenanordnung (55) angeordnet ist.

14. Prothesenliner nach einem der voranstehenden Ansprüche, **dadurch gekennzeichnet, dass** der Grundkörper (10) zumindest teilweise aus einem luftdurchlässigen Material besteht und distal zu der Dichtlippe (20) abgedichtet ist.

15. Prothesenliner nach einem der voranstehenden Ansprüche, **dadurch gekennzeichnet, dass** die Seitenwand (14) in Längserstreckung eine Krümmung aufweist.
